# EUROPÄISCHE PATENTSCHRIFT

(11) **EP 2 866 824 B1**
(45) Veröffentlichungstag und Bekanntmachung des Hinweises auf die Patenterteilung: **10.08.2016**
(21) Anmeldenummer: 13730877.1
(22) Anmeldetag: 19.06.2013
(51) Int. Cl.: A61K 38/19, A61P 11/00

(54) **PHARMAZEUTISCHE ZUSAMMENSETZUNG ZUR BEHANDLUNG DER DURCH SAUERSTOFFARMUT UND VERRINGERTEN LUFTDRUCK VERMITTELTEN PULMONALEN FORM DER HÖHENKRANKHEIT**
PHARMACEUTICAL COMPOUND FOR TREATING THE PULMONARY FORM OF ALTITUDE SICKNESS CAUSED BY OXYGEN DEPRIVATION AND REDUCED AIR PRESSURE
COMPOSITION PHARMACEUTIQUE POUR LE TRAITEMENT DE LA FORME PULMONAIRE DU MAL D'ALTITUDE PROVOQUÉ PAR LE MANQUE D'OXYGÈNE ET LA PRESSION ATMOSPHÉRIQUE RÉDUITE

(30) Priorität: 28.06.2012 EP 12173983
(43) Veröffentlichungstag der Anmeldung: 06.05.2015
(73) Patentinhaber: APEPTICO Forschung und Entwicklung GmbH, 1150 Wien (AT)
(72) Erfinder: FISCHER, Bernhard, A-1160 Wien (AT); LUCAS, Rudolf, Martinez, GA 30907 (US); FISCHER, Hendrik, A-1160 Wien (AT)
(74) Vertreter: Sonn & Partner Patentanwälte
(86) Internationale Anmeldenummer: PCT/EP2013/062777
(87) Internationale Veröffentlichungsnummer: WO 2014/001177

(56) Entgegenhaltungen:
- EP-A1- 2 009 023
- WO-A1-00/09149
- WO-A1-2006/013183
- WO-A1-2009/073909
- WO-A1-2011/085423
- WO-A1-2012/065201
- MAGGIORINI ET AL: "High altitude-induced pulmonary oedema", CARDIOVASCULAR RESEARCH, OXFORD UNIVERSITY PRESS, GB, Bd. 72, Nr. 1, 1. Oktober 2006 (2006-10-01), Seiten 41-50, XP025011558, ISSN: 0008-6363, DOI: 10.1016/J.CARDIORES.2006.07.004 [gefunden am 2006-10-01]

### SEQUENCE LISTING

<110> APEPTICO FORSCHUNG UND ENTWICKLUNG GMBH
<120> Pharmazeutische Zusammensetzung zur Behandlung der durch Sauerstoffarmut und verringerten Luftdruck vermittelten pulmonalen Form der Höhenkrankheit
<130> R 63872
<150> EP 12173983.3
   <151> 2012-06-28
<160> 20
<170> PatentIn version 3.5
<210> 1
   <211> 17
   <212> PRT
   <213> Artificial Sequence
<220>
   <223> synthetisches Peptid
<400> 1
   Cys Gly Gln Arg Glu Thr Pro Glu Gly Ala Glu Ala Lys Pro Trp Tyr
   1               5                   10                  15
   Cys
<210> 2
   <211> 6
   <212> PRT
   <213> Artificial Sequence
<220>
   <223> synthetisches Peptid
<400> 2
   Thr Pro Glu Gly Ala Glu
   1 5
<210> 3
   <211> 14
   <212> PRT
   <213> Artificial Sequence
<220>
   <223> synthetisches Peptid
<400> 3
   Gln Arg Glu Thr Pro Glu Gly Ala Glu Ala Lys Pro Trp Tyr
   1 5 10
<210> 4
   <211> 14
   <212> PRT
   <213> Artificial Sequence
<220>
   <223> synthetisches Peptid
<400> 4
   Pro Lys Asp Thr Pro Glu Gly Ala Glu Leu Lys Pro Trp Tyr
   1 5 10
<210> 5
   <211> 17
   <212> PRT
   <213> Artificial Sequence
<220>
   <223> synthetisches Peptid
<400> 5
   Cys Gly Pro Lys Asp Thr Pro Glu Gly Ala Glu Leu Lys Pro Trp Tyr
   1 5 10 15
   Cys
<210> 6
   <211> 17
   <212> PRT
   <213> Artificial Sequence
<220>
   <223> synthetisches Peptid
<400> 6
   Cys Gly Gln Lys Glu Thr Pro Glu Gly Ala Glu Ala Lys Pro Trp Tyr
   1 5 10 15
   Cys
<210> 7
   <211> 17
   <212> PRT
   <213> Artificial Sequence
<220>
   <223> synthetisches Peptid
<400> 7
   Cys Gly Gln Arg Glu Thr Pro Glu Gly Ala Glu Ala Arg Pro Trp Tyr
   1 5 10 15
   Cys
<210> 8
   <211> 15
   <212> PRT
   <213> Artificial Sequence
<220>
   <223> synthetisches Peptid
<400> 8
   Cys Gly Gln Arg Glu Thr Pro Glu Gly Ala Glu Ala Lys Pro Cys
   1 5 10 15
<210> 9
   <211> 16
   <212> PRT
   <213> Artificial Sequence
<220>
   <223> synthetisches Peptid
<400> 9
   Cys Gln Arg Glu Thr Pro Glu Gly Ala Glu Ala Lys Pro Trp Tyr Cys
   1 5 10 15
<210> 10
   <211> 17
   <212> PRT
   <213> Artificial Sequence
<220>
   <223> synthetisches Peptid
<400> 10
   Cys Gly Gln Arg Glu Thr Pro Glu Gly Ala Glu Ala Lys Phe Trp Tyr
   1 5 10 15
   Cys
<210> 11
   <211> 19
   <212> PRT
   <213> Artificial Sequence
<220>
   <223> synthetisches Peptid
<400> 11
   Lys Ser Pro Gly Gln Arg Glu Thr Pro Glu Gly Ala Glu Ala Lys Pro
   1 5 10 15
   Trp Tyr Glu
<210> 12
   <211> 17
   <212> PRT
   <213> Artificial Sequence
<220>
   <223> synthetisches Peptid
<400> 12
   Lys Gly Gln Arg Glu Thr Pro Glu Gly Ala Glu Ala Lys Pro Trp Tyr
   1 5 10 15
   Gly
<210> 13
   <211> 17
   <212> PRT
   <213> Artificial Sequence
<220>
   <223> synthetisches Peptid
<220>
   <221> MISC_FEATURE
   <222> (1)..(1)
   <223> Orn
<400> 13
   Xaa Gly Gln Arg Glu Thr Pro Glu Gly Ala Glu Ala Lys Pro Trp Tyr
   1 5 10 15
   Gly
<210> 14
   <211> 17
   <212> PRT
   <213> Artificial Sequence
<220>
   <223> synthetisches Peptid
<220>
   <221> MISC_FEATURE
   <222> (1)..(1)
   <223> 4Abu
<400> 14
   Xaa Gly Gln Arg Glu Thr Pro Glu Gly Ala Glu Ala Lys Pro Trp Tyr
   1               5                   10                  15
   Asp
<210> 15
   <211> 17
   <212> PRT
   <213> Artificial Sequence
<220>
   <223> synthetisches Peptid
<220>
   <221> MISC_FEATURE
   <222> (1)..(1)
   <223> beta-Ala
<400> 15
   Xaa Gly Gln Arg Glu Thr Pro Glu Gly Ala Glu Ala Lys Pro Trp Tyr
   1               5                   10                  15
   Glu
<210> 16
   <211> 14
   <212> PRT
   <213> Artificial Sequence
<220>
   <223> synthetisches Peptid
<400> 16
   Thr Lys Pro Ile Glu Leu Gly Pro Asp Glu Pro Lys Ala Val
   1               5                   10
<210> 17
   <211> 17
   <212> PRT
   <213> Artificial Sequence
<220>
   <223> synthetisches Peptid
<400> 17
   Cys Gly Thr Lys Pro Ile Glu Leu Gly Pro Asp Glu Pro Lys Ala Val
   1               5                   10                  15
   Cys
<210> 18
   <211> 15
   <212> PRT
   <213> Artificial Sequence
<220>
   <223> synthetisches Peptid
<400> 18
   Gly Gln Arg Glu Thr Pro Glu Gly Ala Glu Ala Lys Pro Trp Tyr
   1               5                   10                  15
<210> 19
   <211> 17
   <212> PRT
   <213> Artificial Sequence
<220>
   <223> synthetisches Peptid
<400> 19
   Cys Gly Gln Arg Glu Ala Pro Ala Gly Ala Ala Ala Lys Pro Trp Tyr
   1               5                   10                  15
   Cys
<210> 20
   <211> 6
   <212> PRT
   <213> Artificial Sequence
<220>
   <223> synthetisches Peptid
<220>
   <221> MISC_FEATURE
   <222> (2)..(5)
   <223> unbekannt oder sonstige
<400> 20
   Thr Xaa Glu Xaa Xaa Glu
   1               5

## Beschreibung

Die vorliegende Erfindung betrifft die Behandlung der durch Sauerstoffarmut und verringerten Luftdruck vermittelten pulmonalen Form der Höhenkrankheit.

Beim Menschen kann ab einer Höhe von oberhalb 2500 m über dem Meeresspiegel die Höhenkrankheit auftreten. In einer Höhe über 2500 Metern nehmen die Sauerstoffkonzentration und der Luftdruck deutlich ab. Man unterscheidet zerebrale und pulmonale Formen der akuten Höhenkrankheit. Die akute Höhenkrankheit ereignet sich also im Gehirn und auch in der Lunge. Die erste detaillierte klinische Beschreibung der Höhenkrankheit erfolgte im Zuge einer Mont Blanc-Expedition im Jahre 1891. Mindestens vier Mitglieder der Expedition erkrankten an der Höhenkrankheit, wobei ein Mitglied am 2. September 1891 auf einer Höhe von 4000 m starb. Seit diesen Fällen gilt die Höhenkrankheit als individueller lebensbedrohlicher klinischer Zustand.

Unbehandelt kann die pulmonale Form der Höhenkrankheit in weniger als 24 Stunden zum Tod führen, wobei der Tod häufig durch eine sekundäre Lungenembolie eintritt.

Die wirksamste Behandlung aller Formen der akuten Höhenkrankheit ist die Zufuhr von Sauerstoff, zum Beispiele durch schnellen Abstieg des Erkrankten in tiefere Höhenlagen, oder mittels Flaschensauerstoff oder mittels Überdrucksack. Jedoch ist in bergigen Gebieten ein schneller Abstieg oft nicht möglich. Sauerstoffbeatmung durch z.B. Flaschensauerstoff senkt zwar den erhöhten pulmonalarteriellen Druck, normalisiert ihn aber nicht. Auch beim Überdrucksack ist der positive Effekt nur zeitlich begrenzt. Der Therapieerfolg nach Verlassen des Überdrucksackes verschwindet sofort bei Patienten, wenn dieser wieder körperlich aktiv wird.

Eine medikamentöse Behandlung der Höhenkrankheit ist derzeit nur beschränkt und kontroversiell: So wird bei einer schweren Akuten Höhenkrankheit und auch speziell bei der zerebralen Form der Höhenkrankheit Dexamethason vorgeschlagen. Weiters wurde diskutiert, ob PDE-5-Hemmer, die zur Behandlung der primären pulmonal arteriellen Hypertonie eingesetzt werden (Dana Point-Klassifikation 1) auch für die sekundäre pulmonale Hypertonie durch Sauerstoffmangel in Höhen (Dana Point Klassifikation 3) indiziert sind.

Auch natürliche Behandlungsmöglichkeiten für Höhenkrankheit (auch vorbeugend) wurden vorgeschlagen (Tee aus Blättern des Cocastrauchs; Yak-Buttertee; Präparate, die Ginkgo als Wirkstoff enthalten).

Es ist aber festzustellen, dass derzeit die medikamentöse Behandlungsmöglichkeit der pulmonalen Form der Höhenkrankheit noch sehr eingeschränkt ist. Darüber hinaus ist bekannt, dass nur im europäischen alpinen und teilweise auch im nordamerikanischen Bereich mit organisierten Rettungseinsätzen gerechnet werden kann. In abgelegenen Hochgebirgen der Erde und in den extremen Höhen sind Rettungseinsätze und medizinische Hilfe bei Notfällen (mit Einsatz von Flaschensauerstoff oder Überdrucksack) kaum mehr möglich. Die Bereitstellung einer effizienten medikamentösen Behandlung der Höhenkrankheit wäre daher dringend erforderlich, auch als Bestandteil als Notfallpaket für Bergsteiger, die in Gefahr geraten können, die Höhenkrankheit zu entwickeln.

In der EP 2 009 023 A1 werden neue Peptide zur Behandlung von Ödemen vorgeschlagen. Dabei werden diese Peptide mit dem "TEER" ("Transepithelial electrical resistance")-Test unter Verwendung von Calu-3-Zellen bewertet, welcher kein etabliertes Testsystem für Flüssigkeitsentfernung bei Lungenödemen (pulmonary oedema fluid clearance) darstellt. Tatsächlich sind Calu-3-Zellen Bronchialzellen, die nur ca. 1% der zum Gasaustausch dienenden Lungenoberfläche darstellen. Demgegenüber stellen Alveolarzellen 99% der zum Gasaustausch dienenden Lungenoberfläche dar (Hollenhorst et al., J. Biomed. Biotechnol. 2011 (2011), doi:10.1155/2011/174306). Im Gegensatz zum TEER-Test ist als Modell für alvolare Epithelialzellen die humane alvolare Epithelialzelllinie A549 als akzeptierter experimenteller Standard etabliert (Lazrak et al., Am. J. Physiol. Lung Cell. Mol. Physiol. 278 (2000), L848-57).

Aufgabe der vorliegenden Erfindung ist daher, die Möglichkeiten der medikamentösen Behandlung von Patienten mit der pulmonalen Form der Höhenkrankheit deutlich zu verbessern und ein Mittel zur Verfügung zu stellen, mit welchem diese Erkrankung effektiv behandelt, aber auch vermieden werden kann.

Demgemäß betrifft die vorliegende Erfindung ein Peptid, welches aus 7-20, insbesondere 7-17 benachbarten Aminosäuren besteht und das Hexamer TX₁EX₂X₃E umfasst, wobei X₁, X₂ und X₃ jede natürliche oder nicht natürliche Aminosäure sein kann, wobei das Peptid keine TNF-Rezeptor-Bindungsaktivität aufweist und zyklisiert ist, zur Anwendung zur Behandlung und Vermeidung der pulmonalen Form der Höhenkrankheit.

Mit der vorliegenden Erfindung konnte erstmals eine medikamentöse Therapie für die pulmonale Form der Höhenkrankheit zur Verfügung gestellt werden. Daher wurde für die vorliegende Erfindung auch umgehend eine "orphan drug designation" erteilt, und zwar sowohl von der EMA (EMA/OD/144/12) als auch von der US-FDA (12-3829). Dies zeigt das dringende Bedürfnis nach einer Behandlungsmöglichkeit für diese Erkrankung, das durch die vorliegende Erfindung befriedigt wird.

Die erfindungsgemäßen Peptide zur Anwendung sind an sich schon länger bekannt, beispielsweise aus dem europäischen Patent EP 1 264 599 B1, der US 2007/299003 A, WO 94/18325 A1, WO 00/09149 A1, WO 2006/013183 A1 oder WO 2008/148545 A1. Im Zuge der Experimente zur vorliegenden Erfindung wurde nunmehr erkannt, dass sich diese Peptide überraschenderweise auch dazu eignen, die pulmonale Form der Höhenerkrankung zu behandeln, so dass somit erstmals eine einfache und effiziente medikamentöse Behandlungsform für diese Indikation zur Verfügung gestellt werden kann.

Diese - an sich bekannten - Peptide, die erfindungsgemäß zum Einsatz kommen, weisen keine TNF-Rezeptor-Bindungsaktivität auf (Hribar et al., Eur. J. Immunol. 1999; Elia et al., AJRCCM 2003; s. auch: Beispielteil unten) und sind zyklisiert. Bevorzugte Varianten dieser Peptide bestehen aus 7-17 benachbarten Aminosäuren und enthalten das Hexamer TPEGAE (SEQ ID Nr. 2).

Die akute Höhenkrankheit beginnt immer mit subakuter Hypoxie. In der Folge führen Hypoxämie und Hyperkapnie zu Vasodilatation, Hypokapnie zu Vasokonstriktion. In der Höhe ergeben sich nun aus Hypoxämie und Hypokapnie unterschiedliche Effekte: In der Lunge überwiegt Vasokonstriktion, im Gehirn Vasodilatation.

Die Ursache der akuten Höhenkrankheit liegt in einer fehlgeschlagenen Adaptation, vor allem in einer individuell zu geringen Ventilationssteigerung (relative Hypoventilation). Die Folgen sind ausgeprägtere Hypoxämie, höherer Pulmonalarteriendruck, höherer intrakranieller Druck, Flüssigkeitsretention und geringere Erythropoese.

Die pulmonale Form der Höhenkrankheit wird durch Sauerstoffarmut und verringerten Luftdruck vermittelt und ist eine lebensbedrohende Veränderung der Lungenfunktion und kommt vornehmlich in Höhen zwischen 2500 und 6000 m vor. Zwei Drittel aller Fälle ereignen sich zwischen 3000 und 4500 m Seehöhe. Die pulmonale Form der Höhenkrankheit ist die häufigste Todesursache der akuten Höhenkrankheit.

Die pulmonale Form der Höhenkrankheit beginnt oft charakteristischerweise nach Überschreiten der Schwellenhöhe von etwa 2500 m.

Die überschießende, inhomogene, hypoxische Vasokonstriktion in der Lunge führt zu überperfundierten Lungenbezirken mit akuten Infiltraten. Die massiv erhöhte pulmonale Hypertension als Folge einer inhomogenen hypoxischen Vasokonstriktion ist vor allem in peripheren Lungenarealen Ausdruck einer stark erhöhten hypoxisch-pulmonal-vaskularen Reaktion (HPVR, Hypoxic Pulmonary Vascular Response) bei vorher völlig gesunden Menschen. Eine Erhöhung des Pulmonalarteriendruckes ist unter Hypoxie zwar physiologisch, bei der pulmonalen Form der Höhenkrankheit aber deutlich höher ausgeprägt. Allerdings ist die pulmonale Kapillarpermeabilität unter Hypoxie nicht erhöht.

Dies steht im deutlichen Gegensatz zu anderen akuten Lungenerkrankungen, wie zum Beispiele dem Akuten Lungenschaden (ALI), dem Akuten Respiratorischen Distress Syndrom (ARDS) oder dem Hyper-Permeabilitäts-Ödem, welches entweder primär durch direkte Einwirkung einer Noxe oder sekundär als Folgeerscheinung anderer Erkrankungen entstehen kann. Die häufigsten Lungenschädigungen bei ALI, ARDS und beim Hyper-Permeabilitäts- Ödem sind bakterielle und virale Pneumonie, Lungenkontusion, Aspiration von Magensaft, Inhalationstrauma, Rauchgas-Vergiftungen, Beinahe-Ertrinken, massive Bluttransfusionen, Sepsis, Polytrauma, Kardiopulmonaler Bypass oder großflächige Verbrennungen. Bei diesen Lungenerkrankungen steht die Entzündungsreaktion mit einhergehender Schädigung der Alveolarwände im Vordergrund. Dieser Zustand führt zu einer komplexen Aktivierung von pro- und antiinflammatorischen Immunvorgängen die zur entzündlichen Schädigung des Alveolarepithels und des Gefäßendothels führen. Die Folge sind der Verlust an Alveolozyten und Surfactant, das Auftreten eines capillary leaks mit Austritt von Plasmaproteinen und interstitieller Ödembildung. Die entzündlichen Veränderungen sind typischerweise fleckförmig und inhomogen über die gesamte Lunge verteilt. Infiltration, interstitielles und alveoläres Ödem führen letztendlich zu Atelektasen und den klinischen Zeichen der arteriellen Hypoxämie und der pulmonalen Hypertension. Derartige Entzündungsreaktionen haben bei der Höhenerkrankung keine pathologische Bedeutung.

Die Inzidenz einer klinisch manifesten pulmonalen Form der Höhenkrankheit liegt oberhalb von 3500 m bei rund 15%, wobei die Letalität bei 44 % der unbehandelten Patienten liegt.

Die Inzidenz der Höhenkrankheit korreliert nicht mit der VO₂ₘₐₓ, dem Trainingszustand, dem Blutdruck, der Ernährung, dem Zigarettenrauchen oder dem Lebensalter (im Gegensatz zum akuten Lungenschaden (ALI/ARDS), bei dem v.a. Zigarettenrauchen und ein hohes Lebensalter deutliche Risikofaktoren darstellen), wohl aber zum Teil mit der individuellen hypoxischen Atemantwort (HVR) und mit dem Bergziel bzw. der Steiggeschwindigkeit.

Die Unterschiede zwischen der Behandlung der pulmonalen Form der Höhenkrankheit auf der einen Seite und ALI/ARDS auf der anderen Seite wurde auch von den Arzneimittelbehörden EMA und US-FDA bei der Zulassung der vorliegenden Erfindung als "orphan indication" ausdrücklich als Grundlage bei der Prüfung dieser Zulassungen angesehen. Diese Unterscheidung ergibt sich einerseits alleine schon aus dem internationalen Diagnoseklassifikationssystem (ICD) der Weltgesundheitsorganisation WHO: Die pulmonale Form der Höhenkrankheit ist dort unter Kapitel XIX (Verletzungen, Vergiftungen und bestimmte andere Folgen äußerer Ursachen), Krankheitsgruppe T66-T78 (Sonstige und nicht näher bezeichnete Schäden durch äußere Ursachen), Krankheitsklasse T70 (Schäden durch Luft- und Wasserdruck), Subkategorie T70.2 (Sonstige und nicht näher bezeichnete Schäden durch große Höhe) klassifiziert, wohingegen ALI/ARDS in einem völlig anderem Kapitel klassifiziert ist (Kapitel X (Krankheiten des Atmungssystems), Krankheitsgruppe J80-J84 (Sonstige Krankheiten der Atmungsorgane, die hauptsächlich das Interstitium betreffen), Krankheitsklasse J80 (Atemnotsyndrom des Erwachsenen [ARDS])). Der klinische Bereich ist verschieden (Umwelt-, Arbeits- und Sportmedizin für die pulmonale Form der Höhenkrankheit; Anästhesie und Intensivmedizin für ALI/ARDS). Die Ätiologie ist grundlegend verschieden. Die pulmonale Form der Höhenkrankheit entwickelt sich in sonst gesunden Personen ohne zugrunde liegende oder bereits bestehende klinische Zustände durch schnellen, nicht akklimatisierten Aufstieg von gesunden Bergwanderern auf Höhen über 3000 m bzw. Änderungen von Umweltbedingungen, wohingegen ALI/ARDS durch vorangehende klinische Zustände und als Konsequenz einer zugrundeliegenden Pathophysiologie (der Patient leidet bereits an einem anderen, definierbaren klinischen Zustand) bedingt ist, wie: schwere Infektion oder Entzündung, die lokal oder systemisch ist (z.B. bei Sepsis), Aspiration (z.B. durch Magensaft), Einatmen heißer oder giftiger Gase, multiple Bluttransfusionen, Beinaheertrinken, Lungenquetschung, Polytrauma, Verbrennungen, Fettembolie, etc.); ebenso die Pathophysiologie. Bei der pulmonalen Form der Höhenkrankheit führen eine unzureichende ventilatorische Antwort und eine ungewöhnlich starke vasokonstriktorische Reaktion zu Hypoxie wodurch es dann (auch bedingt durch (neurogene) sympathetische Überaktivität) zu erhöhtem Lungendruck, endothelialem Stress und Kaplillaraustritt kommt; bei ALI/ARDS führen Alveolarschäden, Austritt von proteinreicher Flüssigkeit in den Interstitial- und Alveolar-Raum und extensive Freisetzung von Zytokinen und Einwanderung von Neutrophilen zu reduziertem Gasaustausch in der Lunge).

Vor allem unterscheiden sich aber die pulmonale Form der Höhenkrankheit und ALI/ARDS auch bei der Rolle, die entzündliche Prozesse in diesen Erkrankungen haben. ALI/ARDS gehen immer Entzündungsprozesse voraus; diese Entzündungsprozesse spielen eine Hauptrolle in der Pathophysiologie. Demgegenüber spielen entzündliche Prozesse in der pulmonalen Form der Höhenkrankheit so gut wie keine Rolle; sie treten, wenn überhaupt nur als Sekundärmerkmal, nicht jedoch als Ursache für die Erkrankung auf. Während also bei ALI/ARDS eine erhöhte Sekretion von pro-inflammatorischen Modulatoren aus dem Endothel und Neutrophilen, Entzündungsantworten durch Neutrophilen-Aktivierung und Zytokin-Freisetzung, ein hoher Gehalt an Zytokinen und Proteinen in der bronchoalveolaren Lavage-Flüssigkeit (BALF), Anwesenheit von Neutrophilen und Makrophagen in der BALF und durch eine akute Entzündung verursachte erhöhte mikrovaskuläre Lungenpermeabilität deutliche Zeichen der Entzündung darstellen, fehlen diese völlig zumindest in der Anfangsphase der pulmonalen Form der Höhenkrankheit. BALF-Analysen zeigen bei der pulmonalen Form der Höhenkrankheit keine Erhöhung von Leukozyten oder pro-inflammatorischen Modulatoren und keinen Unterschied in surfactant protein A und Clara cell protein.

Schließlich ist auch die Diagnostik der pulmonalen Form der Höhenkrankheit und ALI/ARDS eine völlig andere: Die pulmonale Form der Höhenkrankheit kommt in gesunden, nichtakklimatisierten Bergwanderern vor und entwickelt sich innerhalb von zwei bis fünf Tagen nach Ankunft in großer Höhe. Dabei ist der Druck in den Lungenarterien abnorm erhöht, der Wedge-Druck bleibt aber normal. Bei ALI/ARDS besteht, wie erwähnt, immer ein initiierender klinischer Zustand (z.B. Sepsis). Der Wedge-Druck ist ≤ 18 mmHg, weiters ist in der Regel kein klinisches Anzeichen für linken atrischen Hochdruck (kein erhöhter Druck in der Lungenarterie); das PaO₂/FiO₂-Verhältnis ist ≤ 300 (ALI) im stabilen Zustand.

Die pulmonale Form der Höhenkrankheit und ALI/ARDS sind daher zwei voneinander völlig verschiedene Erkrankungen (Peacock, Eur. Respir. J. 8 (1995), 1819-1821).

Vorzugsweise betrifft die vorliegende Erfindung ein Peptid, welches aus 7-20, insbesondere 7-17 benachbarten Aminosäuren besteht und das Hexamer TPEGAE (SEQ ID Nr. 2) umfasst, wobei das Peptid keine TNF-Rezeptor-Bindungsaktivität aufweist und zyklisiert ist, zur Anwendung zur Behandlung der pulmonalen Form der Höhenkrankheit.

Eine besonders bevorzugte Ausführungsform der vorliegenden Erfindung betrifft ein zyklisiertes Peptid zur Anwendung, bestehend aus einer Sequenz aufeinanderfolgender Aminosäuren, ausgewählt aus der Gruppe bestehend aus
- QRETPEGAEAKPWY (SEQ ID Nr. 3)
- PKDTPEGAELKPWY (SEQ ID Nr. 4)
- CGQRETPEGAEAKPWYC (SEQ ID Nr. 1),
- CGPKDTPEGAELKPWYC (SEQ ID Nr. 5),
- CGQKETPEGAEAKPWYC (SEQ ID Nr. 6),
- CGQRETPEGAEARPWYC (SEQ ID Nr. 7),
- CGQRETPEGAEAKPC (SEQ ID Nr. 8),
- CQRETPEGAEAKPWYC (SEQ ID Nr. 9),
- CGQRETPEGAEAKFWYC (SEQ ID Nr. 10),
- KSPGQRETPEGAEAKPWYE (SEQ ID Nr. 11),
- KGQRETPEGAEAKPWYG (SEQ ID Nr. 12),
- Ornithin-GQRETPEGAEAKPWYG (SEQ ID Nr. 13),
- 4-Aminobutansäure-GQRETPEGAEAKPWYD (SEQ ID Nr. 14),
- β-Alanin-GQRETPEGAEAKPWYE (SEQ ID Nr. 15)
und Fragmenten von mindestens 7 Aminosäuren davon, welche das Hexamer TPEGAE aufweisen, zur Anwendung bzw. zur Herstellung eines Medikaments zur Behandlung der pulmonalen Form der Höhenkrankheit.

Vorzugsweise umfasst das Peptid zur Anwendung die Aminosäuresequenz CGQRETPEGAEAKPWYC (SEQ ID Nr. 1) und ist über die C-Reste zyklisiert. Dieses besonders bevorzugte Peptid zur Anwendung hat somit folgende Aminosäuresequenz (SEQ ID Nr. 1) (NH₂)Cys-Gly-Gln-Arg-Glu-Thr-Pro-Glu-Gly-Ala-Glu-Ala-Lys-Pro-Trp-Tyr-Cys(COOH).

Die Zyklisierung der erfindungsgemäßen Peptide zur Anwendung kann dabei z.B. entweder über eine direkte Zyklisierung über eine Disulfidbrücke zwischen den beiden C-Resten am N- und C-Terminus erreicht werden oder aber indem das Peptid über beide Cysteine an eine Trägersubstanz gekoppelt wird. Dabei werden in den erfindungsgemäßen Peptiden zur Anwendung die Cysteinreste vorzugsweise am Anfang und am Ende des Moleküls vorgesehen. Auch andere funktionelle Gruppen, die eine Zyklisierung des Peptids erreichen, können eingesetzt werden, z.B. indem eine Säuregruppe mit einem Amin oder Alkohol zu einem Amid- oder Ester-Ringschluss führt (hierbei können z.B. die Aminosäuren Asparaginsäure und Glutaminsäure mit Serin, Threonin, Tyrosin, Asparagin, Glutamin oder Lysin, vorzugsweise intramolekular zyklisiert werden). Die Zyklisierung des Peptids erfolgt vorzugsweise durch eine Disulfidbrücke zwischen den C-Resten des Peptids (wenn vorhanden). Es können aber auch auf der Trägersubstanz, insbesondere einem Trägerprotein, Cysteinreste oder andere funktionelle Gruppen vorgesehen werden, die den N-Terminus bzw. den C-Terminus der erfindungsgemäßen Peptide zur Anwendung binden und so die zyklische Natur der erfindungsgemäßen Peptide zur Anwendung gewährleisten.

Insofern ist natürlich auch jegliche Bezugnahme auf ein "erfindungsgemäßes" Peptid zur Anwendung hierin die Bezugnahme auf ein zyklisiertes Peptid.

Die Zyklisierung über Cysteinreste ist besonders bevorzugt, insbesondere entweder über Cysteinreste, die am Anfang und am Ende der erfindungsgemäßen Peptide zur Anwendung vorgesehen sind oder zusätzlich eingeführt sind, und/oder über Cysteinreste an einem Träger, an dem N- und C-Terminus des erfindungsgemäßen Peptids zur Anwendung gekoppelt sind. Besonders bevorzugt ist die intramolekulare Zyklisierung der erfindungsgemäßen Peptide zur Anwendung über die vorgesehenen oder zusätzlich eingeführten Cysteinreste an N- und C-Terminus.

Weitere bevorzugte erfindungsgemäße Peptide zur Anwendung sind daher beispielsweise CGQKETPEGAEAKPWYC (SEQ ID Nr. 6), CGQRETPEGAEARPWYC (SEQ ID Nr. 7), CGQRETPEGAEAKPC (SEQ ID Nr. 8), CQRETPEGAEAKPWYC (SEQ ID Nr. 9) oder CGQRETPEGAEAKFWYC (SEQ ID Nr. 10).

Eine weitere Gruppe bevorzugter erfindungsgemäßer Peptide zur Anwendung sind zyklische Peptide mit einer Sequenz X₁-GQRETPEGAEAKPWY-X₂, wobei X₁ 1 bis 4 Aminosäuren, insbesondere 1 oder 3 Aminosäuren darstellt, diese Aminosäuren natürliche oder unnatürliche Aminosäuren sind, insbesondere X₁ die Aminosäure C, K, Ornithin, 4-Aminobuttersäure, β-Alanin, oder die Sequenz KSP darstellt, X₂ eine natürliche oder unnatürliche Aminosäure sein kann, wobei X₂ insbesondere die Aminosäure C, D, G oder E ist, und wobei X₁ die N-terminale Aminosäure und X₂ die C-terminale Aminosäure ist (GQRETPEGAEAKPWY entspricht SEQ ID Nr. 18). Besonders bevorzugte Beispiele dieser Sequenz X₁-GQRETPEGAEAKPWY-X₂ sind die zyklischen Peptide KSPGQRETPEGAEAKPWYE, KGQRETPEGAEAKPWYG, Ornithin-GQRETPEGAEAKPWYG, 4-Aminobutansäure-GQRETPEGAEAKPWYD, β-Alanin-GQRETPEGAEAKPWYE.

Im zyklischen Peptid KSPGQRETPEGAEAKPWYE sind die Aminosäuren von der C-terminalen Aminosäure Glutaminsäure (E) bis zur N-terminalen Aminosäure Lysin (K) peptidisch verknüpft, während die N-terminale Aminosäure Lysin (K) mit der C-terminalen Aminosäure Glutaminsäure (E) mit Hilfe einer Amidbindung zwischen dem Stickstoff der epsilon-Aminogruppe der Seitenkette des Lysins und dem gamma-Kohlenstoff in der Seitengruppe der Glutaminsäure verbunden ist.

Im zyklischen Peptid KGQRETPEGAEAKPWYG sind die Aminosäuren von der C-terminalen Aminosäure Glycin (G) bis zur N-terminalen Aminosäure Lysin (K) peptidisch verknüpft, während die N-terminale Aminosäure Lysin (K) mit der C-terminalen Aminosäure Glycin (G) mit Hilfe einer Amidbindung zwischen dem Stickstoff der epsilon-Aminogruppe der Seitenkette des Lysins und dem Kohlenstoff der Karboxylgruppe des Glycins verbunden ist.

Im zyklischen Peptid Ornithin-GQRETPEGAEAKPWYG sind die Aminosäuren von der C-terminalen Aminosäure Glycin (G) bis zur N-terminale Aminosäure Ornithin (Orn) peptidisch verknüpft, während die N-terminale Aminosäure Ornithin (Orn) mit der C-terminalen Aminosäure Glycin (G) mit Hilfe einer Amidbindung zwischen dem Stickstoff der delta-Aminogruppe der Seitenkette des Ornithins und dem Kohlenstoff der Karboxylgruppe des Glycins verbunden ist.

Im zyklischen Peptid 4-Aminobutansäure-GQRETPEGAEAKPWYD sind die Aminosäuren von der C-terminalen Asparaginsäure (D) bis zur N-terminalen Aminosäure Glycin (G) peptidisch verknüpft, während die C-terminale Asparaginsäure (D) mit der N-terminalen Aminosäure Glycin mit Hilfe einer Amidbindung zwischen dem Stickstoff der Aminogruppe des N-terminalen Glycin und dem Kohlenstoff C1 der Karboxylgruppe der 4-Aminobuttersäure einerseits, und mit Hilfe einer Amidbindung zwischen dem Stickstoff der Aminogruppe der 4-Aminobuttersäure und dem Kohlenstoff der Karboxylgruppe der Seitenkette der C-terminalen Asparaginsäure andererseits, verbunden ist.

Im zyklischen Peptid β-Alanin-GQRETPEGAEAKPWYE sind die Aminosäuren von der C-terminalen Glutaminsäure (E) bis zur N-terminalen Aminosäure Glycin (G) peptidisch verknüpft, während die C-terminale Glutaminsäure (E) mit der N-terminalen Aminosäure Glycin mit Hilfe einer Amidbindung zwischen dem Stickstoff der Aminogruppe des N-terminalen Glycins und dem Kohlenstoff C1 der Karboxylgruppe des β-Alanin einerseits, und mit Hilfe einer Amidbindung zwischen dem Stickstoff der Aminogruppe des β-Alanin und dem Kohlenstoff der Karboxylgruppe der Seitenkette der C-terminalen Glutaminsäure andererseits verbunden ist.

Die Zyklisierung bei den erfindungsgemäßen Peptiden zur Anwendung kann, wie erwähnt, aber auch durch Bindung des Peptids an Trägersubstanzen erfolgen. Als derartige Zyklisierungs-Trägersubstanzen kommen alle gängigen pharmazeutisch verwendbaren Substanzen in Frage, die in der Lage sind, z.B. mit den SH-Gruppen der Cysteine (oder mit anderen natürlicherweise vorhandenen oder artifiziell eingeführten chemisch reaktiven Gruppen des Peptids) eine kovalente Bindung einzugehen, wobei gängige Trägerproteine, wie Keyhole limpet hemocyanin (KLH), Tetanus-Toxin etc., sich besonders eignen. Auch können am Träger benachbarte bifunktionelle Reste vorgesehen werden (z.B. Säuregruppe neben Amin- oder Alkoholgruppe). In diesem Zusammenhang ist wichtig, dass mit "Zyklisierung" sowohl der intramolekulare Ringschluss als auch die Einbindung eines Trägers umfasst ist (von dem das gebundene Peptid hervorragt (indem der N- und der C-Terminus des Peptids an den Träger gebunden ist)), wobei das derart zyklisierte Peptid die zyklische Raumstruktur zeigt und entsprechend stabilisiert ist.

Eine Gruppe besonders bevorzugter erfindungsgemäßer Peptide zur Anwendung ist daher die Gruppe mit den SEQ ID Nrn. 1 und 5 bis 15).

Die erfindungsgemäßen Peptide zur Anwendung weisen insbesondere eine aktivierende Wirkung auf den Amilorid-sensitiven epithelialen Natrium-Ionenkanal (ENaC). Diese Eigenschaft kann vorteilhafter Weise mit der Methodologie gemäß Eaton et al., (Fed. Proc. 45 (1986), 2707) und Hamill et al. (Pflugers Arch. 391 (1981), 85-100), wie im Beispielteil dargelegt, geprüft werden.

Bevorzugter Weise wird das erfindungsgemäße Peptid zur Anwendung zur Behandlung der pulmonalen Form der Höhenkrankheit in einer pharmazeutischen Zusammensetzung zur Anwendung zur Verfügung gestellt, die einen pharmazeutisch akzeptablen Träger umfasst. Dabei ist die pharmazeutische Zusammensetzung vorzugsweise in einer Form hergerichtet, die zur Verabreichung am Menschen geeignet ist.

Der Ausdruck "eine pharmazeutische Zusammensetzung" bezieht sich auf jede Zusammensetzung, die ein Peptid, wie oben definiert, umfasst (natürlich auch geeignete (i.e. nicht miteinander negativ interferierende) Gemische des erfindungsgemäßen Peptids zur Anwendung mit weiteren Wirksubstanzen; es ist jedoch bevorzugt, das erfindungsgemäße Peptid zur Anwendung als einzige Wirksubstanz vorzusehen, welche die hierin beschriebenen Zustände verhindert, verbessert oder heilt. Insbesondere bezieht sich der Ausdruck "eine pharmazeutische Zusammensetzung" auf eine Zusammensetzung, die ein Peptid, wie oben beschrieben, und einen pharmazeutisch akzeptablen Träger oder Exzipienten (beide Ausdrücke können austauschbar verwendet werden) aufweist. Geeignete Beispiele von Trägern oder Exzipienten, die dem Fachmann bekannt sind, sind Wasser, Kochsalzlösung, Natriumphosphat, Natriumacetat, Natriumcarbonat, Zitrat, Glycin, Glycylglycin, Histidin, Lysin, Arginin, TRIS und Natriumcitrat oder Mischungen davon. Natürlich können auch Ringer-Lösung, Dextrose-Lösung oder Lösungen nicht reduzierbarer Zucker eingesetzt werden; demgemäß eignen sich auch Mannit, Trehalose, Saccharose, Sorbit, Fruchtzucker, Maltose, Lactose oder Dextran, Hank-Lösung, fixierte Öle, Ethyloleat, 5% Dextrose in Kochsalzlösung, Substanzen, die die Isotonie und die chemische Stabilität verbessern, Puffer und Konservierungsmittel als derartige Träger. Andere geeignete Träger inkludieren jeden Träger, der nicht selbst die Produktion von Antikörpern, die für das die Zusammensetzung erhaltende Individuum schädlich sind, induziert, wie Proteine, Polysaccharide, Polymilchsäuren, Polyglykolsäuren, polymere Aminosäuren und Aminosäure-Copolymere. Bei der Formulierung der erfindungsgemäßen pharmazeutischen Zusammensetzung sind natürlich die einschlägigen Richtlinien (z.B. die (europäische oder US) Pharmakopöe) zu beachten. Dabei kann das in der erfindungsgemäßen Zusammensetzung zur Anwendung vorgesehene Peptid, wie erwähnt, auch durch direkte kovalente Bindung an diese Träger zyklisiert werden.

Die erfindungsgemäße pharmazeutische Zusammensetzung zur Anwendung kann (als Medikament) mit jedem im Wissen des Fachmanns liegenden geeigneten Verfahren verabreicht werden, insbesondere ist bevorzugt, das erfindungsgemäße Peptid zur Anwendung bzw. die erfindungsgemäße Zusammensetzung zur Anwendung in die Lunge zu verabreichen. Der bevorzugte Verabreichungsweg ist Inhalation (durch Aerosole) aber auch die intravenöse Gabe, Instillation, orale Gabe oder Kombinationen daraus. Bei inhalativer, parenteraler oder oraler Verabreichung wird das Medikament dieser Erfindung in Dosiseinheitsform, wie als Lösung, Suspension oder Emulsion, in Verbindung mit den oben definierten pharmazeutisch akzeptablen Exzipienten formuliert. Die Dosierung und Verabreichungsart kann jedoch natürlich auch im Einzelfall vom jeweiligen Individuum abhängen.

Dabei wird die jeweils erforderliche wirksame Menge an das Individuum, das die Verabreichung benötigt, verabreicht. Dabei ist die "wirksame Menge" als eine Menge zu verstehen, die wirksam genug ist, um den beabsichtigten therapeutischen oder prophylaktischen Effekt zu erzielen, also z.B. eine weitere Verschlechterung der Erkrankung zu verhindern oder wirksam zu behandeln. Dabei wird meist von einem durchschnittlichen Patienten ausgegangen, jedoch können die tatsächlichen wirkungsvollen Mengen der Komponenten in der Zusammensetzung so formuliert werden, dass die Art der Verabreichung und das Alter, Gewicht, Zustand des Patienten und Grad und Fortschritt der Erkrankung berücksichtigt werden (z.B. mittels eines passenden, herkömmlichen pharmakologischen Protokolls).

Vorzugsweise ist daher der pharmazeutisch akzeptable Träger in der erfindungsgemäßen Zusammensetzung zur Anwendung ausgewählt aus Wasser (besonders bevorzugt: Wasser für Injektion), Kochsalz, Natriumphosphat, Natriumacetat, Natriumcarbonat, Zitrat, Glycin, Glycylglycin, Histidin, Lysin, Arginin, TRIS, Natriumcitrat, Ringer-Lösung, Dextrose, Mannit, Trehalose, Saccharose, Sorbit, Fruchtzucker, Maltose, Lactose oder Dextran, Hank-Lösung, fixierte Öle, Ethyloleat, Substanzen, die die Isotonie und die chemische Stabilität verbessern, Konservierungsmittel, pharmazeutisch akzeptable Proteine, Polysaccharide, Polymilchsäuren, Polyglykolsäuren, polymere Aminosäuren und Aminosäure-Copolymere.

Das Medikament kann beispielsweise so verabreicht werden, dass das Peptid zur Anwendung der vorliegenden Erfindung in einer Dosis von zwischen 1 µg/kg und 10 mg/kg, mehr bevorzugt zwischen 10 µg/kg und 5 mg/kg, am meisten bevorzugt zwischen 0,1 und 2 mg/kg, gegeben wird. Vorzugsweise wird es als Bolus-Dosis gegeben. Es kann aber auch eine kontinuierliche Inhalation oder Infusion oder eine Verabreichung mittels wiederholter Gabe verwendet werden.

Besonders bevorzugte erfindungsgemäße Zusammensetzungen zur Anwendung enthalten das Peptid in einer Menge von 1 µg bis 10 g, vorzugsweise von 10 µg bis 1 g, insbesondere von 1 mg bis 100 mg.

Besonders bevorzugte erfindungsgemäße Zusammensetzungen zur Anwendung in flüssiger Form enthalten das Peptid in einer Menge von 1 µg bis 10 g, vorzugsweise von 10 µg bis 1 g, insbesondere von 1 mg bis 100 mg, und liegen in einem Volumen von 0,5 bis 10 ml, insbesondere in einem Volumen von 1 bis 5 ml, vor.

Die erfindungsgemäße Zusammensetzung zur Anwendung kann vorzugsweise auch in Trockenform mittels Pulverinhalator verabreicht werden. Beispiele für derartige Pulverinhalatoren, die für die vorliegende Erfindung eingesetzt werden können, sind in den US-Patenten 4.995.385 und 4.069.819 beschrieben; bereits etablierte Produkte sind SPINHALER^{®}, ROTAHALER^{®}, FLOWCAPS^{®}, INHALATOR^{®}, DISKHALER^{®} und AEROLIZER^{®}.

Die erfindungsgemäße Zusammensetzung zur Anwendung kann vorzugsweise auch als Aerosol mittels Flüssigkeitsvernebler verabreicht werden. Beispiele für derartige Flüssigkeitsvernebler sind etablierte Produkte wie Aeroneb^{®} und Pari^{®}.

Gemäß einer bevorzugten Ausführungsform ist die erfindungsgemäße Zusammensetzung zur Anwendung dadurch gekennzeichnet, dass das Peptid in einer vernebelbaren Pulverformulierung oder in einer vernebelbaren Flüssigformulierung vorliegt.

Die Erfindung wird anhand der nachfolgenden Beispiele und der Zeichnungsfiguren, auf die sie aber selbstverständlich nicht beschränkt ist, näher erläutert.

Es zeigen:

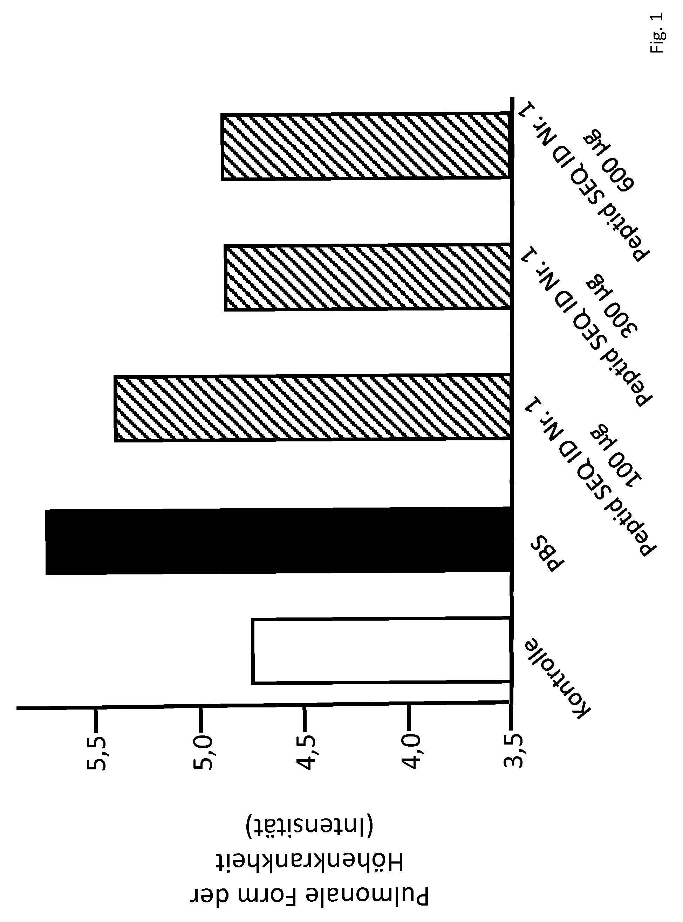

Figur 1: Die Intensität der pulmonalen Form der Höhenkrankheit in Ratten wurde 4 Stunden nach intratrachealer Gabe von Kochsalzlösung bzw. Peptid SEQ ID Nr. 1 bestimmt. Kontrolle: Kontroll-Ratten unter Bedingungen von normalen Sauerstoff- und Luftdruckwerten. PBS: Ratten unter Bedingungen von verringertem Sauerstoff und Luftdruck und intratrachealer Gabe von Kochsalzlösung. Peptid SEQ ID Nr. 1 100 µg: Ratten unter Bedingungen von verringertem Sauerstoff und Luftdruck und intratrachealer Gabe von 100 µg Peptid SEQ ID Nr. 1. Peptid SEQ ID Nr. 1 300 µg: Ratten unter Bedingungen von verringertem Sauerstoff und Luftdruck und intratrachealer Gabe von 300 µg Peptid Seq. ID No. 1. Peptid SEQ ID Nr. 1 600 µg: Ratten unter Bedingungen von verringertem Sauerstoff und Luftdruck und intratrachealer Gabe von 600 µg Peptid SEQ ID Nr. 1.

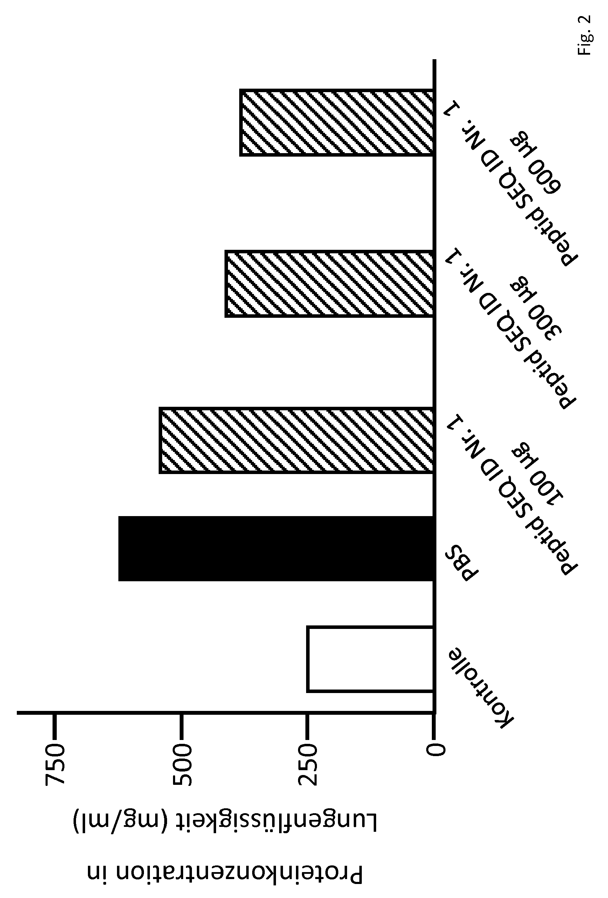

Figur 2: Der Gehalt an Protein in der Lungenflüssigkeit in Ratten wurde 4 Stunden nach intratrachealer Gabe von Kochsalzlösung bzw. Peptid SEQ ID Nr. 1 bestimmt. Kontrolle: Kontroll-Ratten unter Bedingungen von normalen Sauerstoff- und Luftdruckwerten. PBS: Ratten unter Bedingungen von verringertem Sauerstoff und Luftdruck und intratrachealer Gabe von Kochsalzlösung. Peptid SEQ ID Nr. 1 100 µg: Ratten unter Bedingungen von verringertem Sauerstoff und Luftdruck und intratrachealer Gabe von 100 µg Peptid SEQ ID Nr. 1. Peptid SEQ ID Nr. 1 300 µg: Ratten unter Bedingungen von verringertem Sauerstoff und Luftdruck und intratrachealer Gabe von 300 µg Peptid SEQ ID Nr. 1. Peptid SEQ ID Nr. 1 600 µg: Ratten unter Bedingungen von verringertem Sauerstoff und Luftdruck und intratrachealer Gabe von 600 µg Peptid SEQ ID Nr. 1.

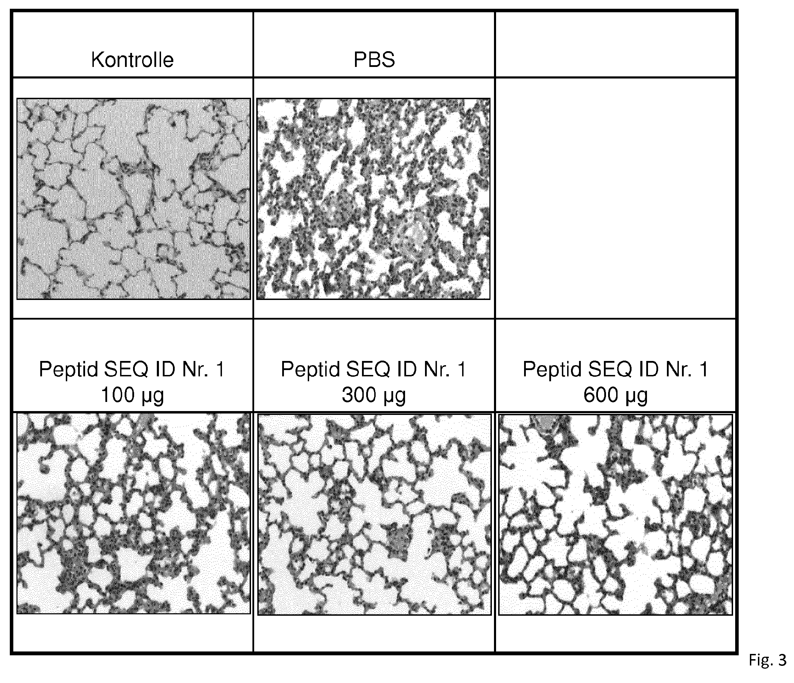

Figur 3: Histologisches Erscheinungsbild von Lungengewebe in Ratten 4 Stunden nach intratrachealer Gabe von Kochsalzlösung bzw. Peptid SEQ ID Nr. 1. Kontrolle: Kontroll-Ratten unter Bedingungen von normalen Sauerstoff- und Luftdruckwerten. PBS: Ratten unter Bedingungen von verringertem Sauerstoff und Luftdruck und intratrachealer Gabe von Kochsalzlösung. Peptid SEQ ID Nr. 1 100 µg: Ratten unter Bedingungen von verringertem Sauerstoff und Luftdruck und intratrachealer Gabe von 100 µg Peptid Seq. ID No. 1. Peptid SEQ ID Nr. 1 300 µg: Ratten unter Bedingungen von verringertem Sauerstoff und Luftdruck und intratrachealer Gabe von 300 µg Peptid SEQ ID Nr. 1. Peptid SEQ ID Nr. 1 600 µg: Ratten unter Bedingungen von verringertem Sauerstoff und Luftdruck und intratrachealer Gabe von 600 µg Peptid SEQ ID Nr. 1.

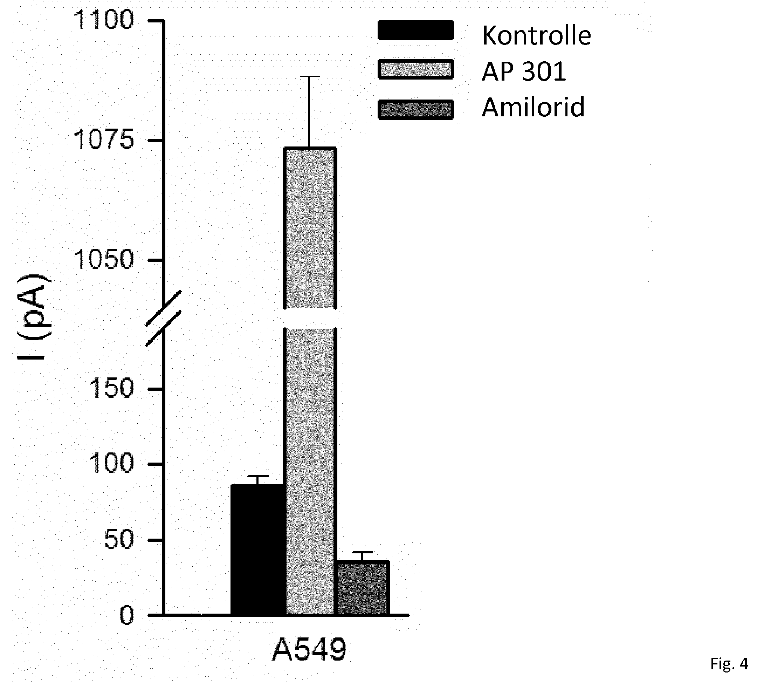

Figur 4: Mittelwerte der nach innen fließenden Na⁺-Ströme in A549-Zellen, in einem Ganzzell-Patch-Clamp-Test während der Kontrollphase bei -100 mV geclamped, nach Zugabe von PEPTID SEQ ID NR. 1 ("AP301") (240 nM) und nach Zugabe von Amilorid (100 mM) zur Badlösung. Die Werte sind Mittelwerte +/- SE.

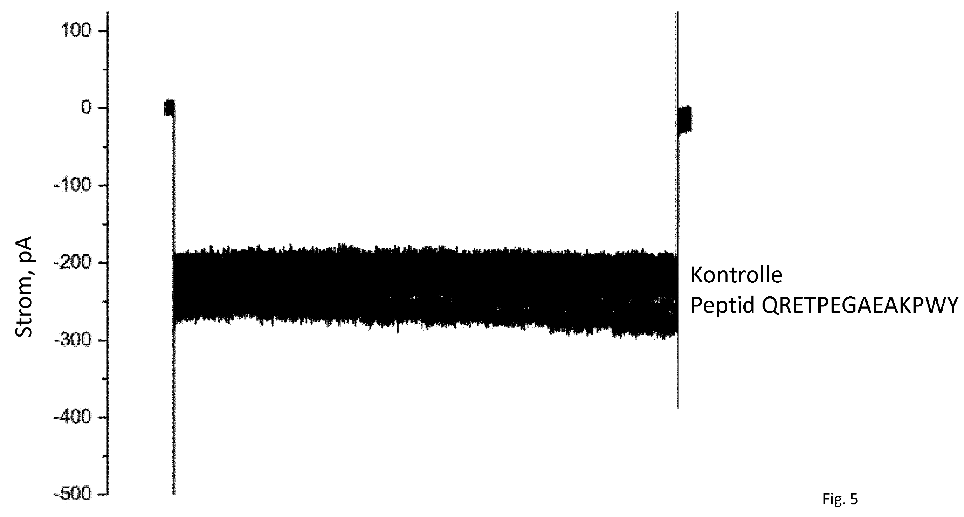

Figur 5: Wirkung des synthetischen Peptids QRETPEGAEAKPWY (SEQ ID Nr.3, im Stand der Technik als geeignet zur Behandlung von Ödemen beschrieben, ist jedoch, im Gegensatz zur erfindungsgemäßen Form, bei diesem Experiment nicht zyklisiert) auf den Na⁺-Strom in einer A549 Zelle gepatcht im Ganzzell-Modus. Repräsentative Originalaufzeichnung einer Zelle geclamped bei einem Haltepotential von -100 mV während der Kontrollphase und nach Zugabe des Peptids QRETPEGAEAKPWY (300 nM) in der Bad-Lösung.

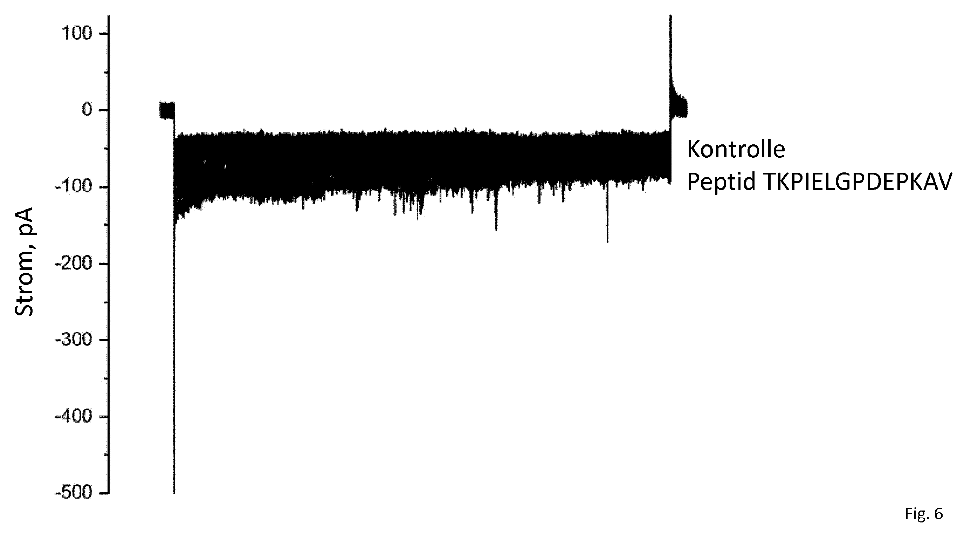

Figur 6: Wirkung des synthetischen Peptids TKPIELGPDEPKAV (SEQ ID Nr. 16; im Stand der Technik als geeignet zur Behandlung von Ödemen beschrieben, ist jedoch im Gegensatz zu den erfindungsgemäßen Peptiden nicht zyklisiert und enthält nicht die Kernsequenz TX₁EX₂X₃E bzw. TPEGAE) auf den Na⁺-Strom in einer A549 Zelle gepatcht im Ganzzell-Modus. Repräsentative Originalaufzeichnung einer Zelle geclamped bei einem Haltepotential von -100 mV während der Kontrollphase und nach Zugabe des Peptids TKPIELGPDEPKAV (300 nM) in der Bad-Lösung.

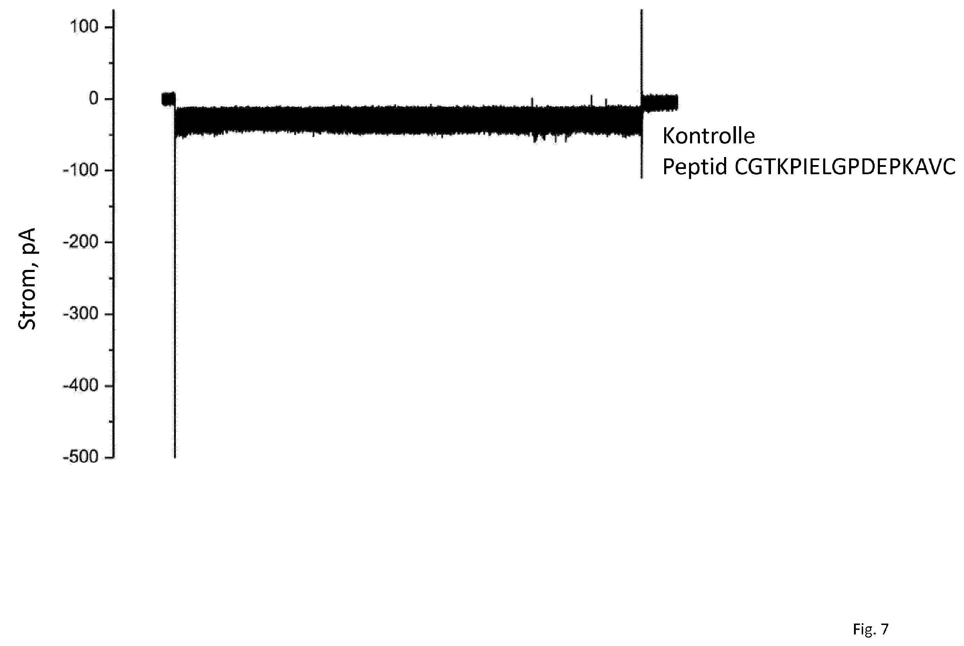

Figur 7: Wirkung des synthetischen zyklischen Peptids CGTKPIELGPDEPKAVC (SEQ ID Nr. 17; im Stand der Technik als geeignet zur Behandlung von Ödemen beschrieben, enthält jedoch im Gegensatz zu den erfindungsgemäßen Peptiden nicht die Kernsequenz TX₁EX₂X₃E bzw. TPEGAE) auf den Na⁺-Strom in einer A549 Zelle gepatcht im Ganzzell-Modus. Repräsentative Originalaufzeichnung einer Zelle geclamped bei einem Haltepotential von -100 mV während der Kontrollphase und nach Zugabe des zyklischen Peptids CGTKPIELGPDEPKAVC (300 nM) in der Bad-Lösung.

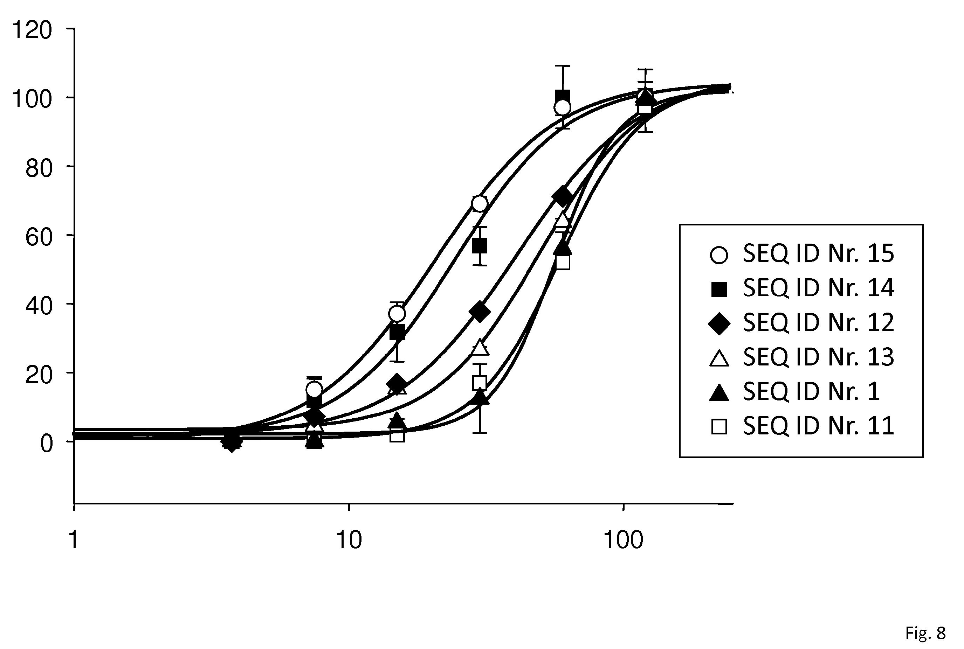

Figur 8: Aktivität der zyklischen Peptide SEQ ID Nrn. 1 und 11 bis 15 in Abhängigkeit der Konzentration. Auf der x-Achse ist die Konzentration in einer logarithmischen Skala in nM aufgetragen; auf der y-Achse ist der Natrium-Ionenstrom (in %) aufgetragen.

Beispiel 1:

### Verwendung des erfindungsgemäßen Peptids mit SEQ ID Nr. 1 zur Anwendung zur Behandlung der pulmonalen Form der Höhenkrankheit

Mit dem vorliegenden Beispiel wird in einem experimentellen Rattenmodell der Höhenkrankheit gezeigt, dass die erfindungsgemäße Zielstellung erreicht wurde, indem an der pulmonalen Form der Höhenkrankheit leidenden Ratten das erfindungsgemäße synthetische Peptid (SEQ ID Nr. 1) verabreicht wurde. Körperliche Anstrengung unter Bedingungen von verringertem Sauerstoff und Luftdruck, wie sie in großen Höhen vorkommen, sind die 2 Hauptfaktoren, die zur Entwicklung der pulmonalen Form der Höhenkrankheit führen. Daher simuliert das gewählte Rattenmodell, in welchem die Ratten unter Bedingungen von verringertem Sauerstoff und verringertem Luftdruck körperliche Tätigkeit verrichteten, einen körperlich anstrengenden Aufstieg auf große Höhen. Dies geschieht ohne eine vorhergehende Akklimatisation vorzunehmen. Dies entspricht dem Szenario, wie es bei Bergsteigern anzutreffen ist, die in großen Höhen an der pulmonalen Form der Höhenkrankheit leiden. Im verwendeten Model entwickeln die Ratten die für die pulmonale Form der Höhenkrankheit typischen Symptome, wie an der "Intensität der pulmonalen Form der Höhenkrankheit", der erhöhten Proteinkonzentration in der Lungenflüssigkeit und dem histologischen Erscheinungsbild des Lungengewebes ablesbar ist. Es ist weiter anzumerken, dass der Lungenschaden in diesem Modell nicht durch Verabreichung von Endotoxinen, Mikroben oder anderen die Lunge schädigenden Agenzien verursacht wird. Eine verstärkte Inflammation der Lunge tritt nicht auf. Es wurde auch kein spezieller Rattenstamm für dieses Experiment verwendet. Daher ist dieses Rattenmodell gut geeignet ein Medikament zur Behandlung der pulmonalen Form der Höhenkrankheit zu untersuchen.

### Methode

Laborratten (Sprague Dawley Ratten) verrichteten durch externe Stimulation 48 Stunden lang körperliche Tätigkeit unter Bedingungen von verringertem Sauerstoff und Luftdruck. Hier wurde der Luftdruck auf einen Wert unter 430 Torr reduziert, sodass eine Höhe von über 4500 m simuliert wurde. Eine vorhergehende Akklimatisation der Ratten an die Höhe von über 4500 m wurde nicht vorgenommen. Während dieser Zeit konnten die Ratten alle 4 Stunden eine 15-20 minütige Pause einlegen um Wasser und Nahrung aufzunehmen. Nach 48 h körperlicher Tätigkeit auf der simulierten Höhe von über 4500 m wurden die Ratten intratracheal mit 300 µl/Versuchstier Peptid SEQ ID Nr. 1 (100 µg, 300 µg und 600 µg) oder 300 µl Kochsalzlösung behandelt. Anschließend verbrachten die Ratten weitere 4 Stunden unter Bedingungen von verringertem Sauerstoff und Luftdruck auf der simulierten Höhe von über 4500 m. Dann wurden die Lungen entnommen und die Intensität der pulmonalen Form der Höhenkrankheit ermittelt (Figur 1), der Gehalt an Protein in der Lungenflüssigkeit ermittelt (Figur 2) und das histologische Erscheinungsbild des Lungengewebes bestimmt (Figur 3).

### Ergebnis

Die Untersuchung ergab, dass die intratracheale Gabe von Peptid SEQ ID Nr. 1 an Laborratten, welche den Bedingungen von verringertem Luftdruck und verringerter Sauerstoffkonzentration ausgesetzt waren, zu Verringerung der Intensität der pulmonalen Form der Höhenkrankheit führte (Figur 1). Dies konnte für 100 µg/Laborratte und 600 µg/Laborratte und vorzugsweise für 300 µg/Laborratte Peptid SEQ ID Nr. 1 nachgewiesen werden.

Die Untersuchung ergab weiters, dass die intratracheale Gabe von Peptid SEQ ID Nr. 1 an Laborratten, welche den Bedingungen von verringertem Luftdruck und verringerter Sauerstoffkonzentration ausgesetzt waren, zu Verringerung der Proteinkonzentration in der Lungenflüssigkeit führte (Figur 2). Dies konnte für 100 µg/Laborratte und 600 µg/Laborratte und vorzugsweise für 300 µg/Laborratte Peptid SEQ ID Nr. 1 nachgewiesen werden.

Die histologische Untersuchung ergab, dass die mit Kochsalzlösung behandelten Ratten geschwollenes Lungengewebe mit roten Blutkörperchen aufwiesen, wobei das Lungengewebe in Ratten nach Gabe von Peptid SEQ ID Nr. 1 vergleichbar mit gesundem Lungengewebe der Kontroll-Ratten war, die nicht den Bedingungen von verringertem Sauerstoff und Luftdruck ausgestzt waren.

### Beispiel 2:

Ex vivo Beurteilung der pro-inflammatorischen Eigenschaften des erfindungsgemäßen Peptids mit SEQ ID Nr. 1 im menschlichen Vollblut.

Eine pharmakologische ex-vivo-Sicherheitsstudie hinsichtlich des erfindungsgemäßen Peptids zur Anwendung SEQ ID Nr. 1 im menschlichen Vollblut wurde durchgeführt, um festzustellen, ob das Peptid SEQ ID Nr. 1 zur Freisetzung des pro-inflammatorischen Markers Interleukin-6 (IL-6) aus frischem Vollblut führt (d.h. ob Peptid SEQ ID Nr. 1 TNFspezifische entzündliche Aktivität (i.e. TNF-Rezeptor-Bindungsaktivität) zeigt oder nicht). In dieser Studie ist frisches Vollblut verwendet worden, hierbei handelt es sich um ein anerkanntes Vorhersagemodell für die Beurteilung der Entzündungsreaktion in vivo.

### Zusammenfassung der Methodik

Es war das Ziel dieser Studie, die proinflammatorische Signal-Kapazität des Peptids SEQ ID Nr. 1 zu bestimmen. Dabei wurden Vollblut-Kulturen verwendet und die Sekretion von Interleukin-6 (IL-6), ein sehr sensitiver Marker für proinflammatorische Stimulation, mittels ELISA quantifiziert.
- Testsystem: 25 ml frisch entnommenes heparinisiertes Blut von 5 gesunden Probanden (GP) wurde in den Tests verwendet.
- Prüfgegenstand Identifikation:: Peptid SEQ ID Nr. 1 (Dosis: 1 ng/ml bis 10 µg/ml; einmalige Verabreichung in Lösung)
- Beschreibung:: Weißes Pulver, Reinheit 96%

### Vollblut-Kulturen

Vollblut (VB)-Kulturen wurden durch Pipettieren von 1 ml VB in Vertiefungen von 24-Näpfchen-Platten durchgeführt. In jedem Experiment wurden unstimulierte und stimulierte Kontroll-Kulturen inkludiert.

Wenn möglich wurden die zu untersuchenden Substanzen und Stimulanzien immer in gleichem Volumen in jedem Näpfchen in einem gegebenen Experiment verwendet, welches nicht größer als 10% des Gesamtvolumens in einem Näpfchen ist. Unstimulierte Kontrollen erfolgten mit PBS. Volumeneinstellung und Verdünnungen für verschiedene Behandlungen wurden ebenfalls mit PBS durchgeführt.

Der Inhalt jedes Näpfchens wurde gemischt und die Platten bei 37°C und 5% CO₂ für 24 Stunden inkubiert. Nach der Inkubation wurde der Inhalt jedes Näpfchens in ein frisches 1,5 ml Mikroröhrchen überführt und bei 8000 bis 9000 x g für 15 Minuten zentrifugiert. Der Überstand jeder Probe wurde einzeln auf zwei 1,5 ml Reaktionsgefäße aufgeteilt und bei -20°C bis zum Gebrauch gelagert.

### Nachweis von Interleukin-6

Interleukin-6 wurde mittels eines spezifischen ELISA quantifiziert (Human IL-6 ELISA-Set, BD Biosciences, Cat. Nr. 555220) unter Einsatz eines Anti-Human-IL-6 Antikörpers als Fänger-Antikörper, eines biotinylierten anti-human IL-6 Detektionsantikörpers, Avidin-Meerrettichperoxidase-Konjugat als Enzym-Reagenz und rekombinantem IL-6 als Standard. Absorptionsmessung bei 450 nm wurde mit dem Packard FusionReader durchgeführt.

### Datenanalyse

Die Ergebnisse jeder Platte wurden gespeichert und mit der FusionDataAnalysis Software ausgewertet.

### Zusammenfassung der Ergebnisse der Studie

Es war das Ziel dieser Studie, die pro-inflammatorische Signalisierungs-Kapazität des Peptids SEQ ID Nr. 1 zu bestimmen. Vollblut-Kulturen wurden verwendet und die Sekretion von IL-6, einem sehr sensitiven Marker für entzündliche Pro-Stimulation, wurde mittels ELISA quantifiziert.

Vollblutproben von fünf gesunden Probanden wurden entweder unstimuliert belassen (negative Kontrolle), stimuliert mit hohen und niedrigen Dosen von LPS (positive Kontrollen) oder mit dem Peptid in neun halblogarithmischen Verdünnungen von 10 µg/ml bis 1 ng/ml inkubiert. Die Ergebnisse sind in der nachfolgenden Tabelle dargestellt:

**Tabelle: Freisetzung von Interleukin-6 aus frischem Vollblut bei Zugabe von Peptid SEQ ID Nr. 1 und LPS**

| | Peptid SEQ ID Nr. 1 | Positive Kontrolle (LPS) |
|---|---|---|
| Konzentration | Konzentration von IL-6 (pg/ml, n = 5) | |
| 0 (Negativkontrolle) | weniger als 0,5 | weniger als 0,5 |
| 10 mg/ml | weniger als 0,5 | 195,640 |
| 1 mg/ml | weniger als 0,5 | 108,370 |
| 3 ng/ml | weniger als 0,5 | 34,867 |
| 1 ng/ml | weniger als 0,5 | nicht bestimmt |

Die Ergebnisse zeigen deutlich, dass das Peptid SEQ ID Nr. 1 keine nachweisbare Menge von IL-6-Sekretion bei keinem der getesteten Konzentrationen induzierte. Die positiven Kontrollen (LPS) führten zu einer starken Induktion der IL-6-Sekretion.

### Diskussion

Die Versuche wurden durchgeführt, um festzustellen, ob das Peptid SEQ ID Nr. 1 die Induktion einer pro-inflammatorischen Kaskade vermittelt. Readout-Parameter war die induzierte Sekretion von IL-6 in Vollblut-Kulturen aus fünf gesunden Spendern. Die Ergebnisse zeigten deutlich, dass das Peptid SEQ ID Nr. 1 kein nachweisbares Niveau von IL-6 in den Spenderkulturen induzierte. Damit ist bewiesen, dass das Peptid SEQ ID Nr. 1 keine pro-inflammatorische Antwort in dem gewählten ex vivo-Modell induziert und somit keine TNF-Rezeptor-Bindungsaktivität aufweist. Dieser Test kann für beliebige Varianten des erfindungsgemäßen Peptids zur Anwendung angewendet werden, um das Merkmal der Freiheit von TNF-Rezeptor-Bindungsaktivität festzustellen.

Beispiel 3: Beurteilung der Bioaktivität des erfindungsgemäßen Peptids zur Anwendung im Vergleich zur nicht-zyklisierten (und somit nicht erfindungsgemäßen) Form des Peptids und anderen synthetischen Peptiden, die im Stand der Technik zur Behandlung von Ödemen vorgeschlagen worden sind, in einem Patch-Clamp-Assay mit A549 Zellen

### Zusammenfassung:

In diesem Beispiel wurde die biologische Aktivität des erfindungsgemäßen Peptids zur Anwendung mit drei anderen synthetischen Peptiden im Hinblick auf die Fähigkeit zur Induktion des Natrium-Stroms beurteilt. Die synthetischen Vergleichspeptide wurden auch in der europäischen Patentanmeldung EP 2 009 023 A1 als Peptide zur Behandlung von Ödemen vorgeschlagen. Für diese Peptide wurde in der EP 2 009 023 A1 unterstellt, sie würden in der Lage sein, die Akkumulation von überschüssiger Flüssigkeit in Geweben zu inhibieren oder zu reduzieren. In der EP 2 009 023 A1 wurde diese Eigenschaft anhand des TEER-Tests untersucht; im Rahmen des vorliegenden Beispiels wird diese biologische Aktivität in einem Ganzzell-Patch-Clamp-Test mit A549-Zellen untersucht.

Dieses Messprinzip (Ganzzell-Patch-Clamp-Test) spiegelt den Flüssigkeitshaushalt in der menschlichen Lunge bedeutend besser wider und ist daher ein anerkanntes Testsystem für diese Fragestellung. Der Flüssigkeitshaushalt in der gesunden erwachsenen menschlichen Lunge hängt von Ionentransportmechanismen ab, die über das Lungenepithel führen, wobei die Beteiligung von Na⁺-Transporter bei der Clearance von Alveolarflüssigkeit in vielen Studien dokumentiert worden ist. Insbesondere wurde dabei der Amilorid-sensitive epitheliale Natrium-Ionenkanal (ENaC) der Typ II-Alveolarzellen als Hauptregulator der Clearance von Alveolarflüssigkeit identifiziert.

Um die Aktivität des Amilorid-sensitiven epithelialen Natrium-Ionenkanal (ENaC) zu beurteilen und dessen Aktivierung durch biologische und chemische Verbindungen zu bestimmen, wurde die Ganzzell-Patch-Clamp-Technik als die experimentelle Methodik der Wahl zur Messung der Natrium-Ionenbewegung über die apikale Membran von Alveolarzellen zur Vorhersage der Clearance von Alveolarflüssigkeit etabliert.

Demgemäß wurde im vorliegenden Beispiel die biologische Aktivität des erfindungsgemäßen Peptids zur Anwendung und drei synthetischer Peptide, QRETPEGAEAKPWY (SEQ ID Nr.3, im Stand der Technik als geeignet zur Behandlung von Ödemen beschrieben, ist jedoch, im Gegensatz zur erfindungsgemäßen Form, bei diesem Experiment nicht zyklisiert), TKPIELGPDEPKAV (SEQ ID Nr. 16; im Stand der Technik als geeignet zur Behandlung von Ödemen beschrieben, ist jedoch im Gegensatz zu den erfindungsgemäßen Peptiden zur Anwendung nicht zyklisiert und enthält nicht die Kernsequenz TX₁EX₂X₃E bzw. TPEGAE) und CGTKPIELGPDEPKAVC (SEQ ID Nr. 17; im Stand der Technik als geeignet zur Behandlung von Ödemen beschrieben, enthält jedoch im Gegensatz zu den erfindungsgemäßen Peptiden zur Anwendung nicht die Kernsequenz TX₁EX₂X₃E bzw. TPEGAE) mittels Ganzzell-Patch-Clamp-Messungen auf A549-Zellen, eine kontinuierliche Zelllinie von humanen Alveolar-Typ II-Zellen, bestimmt.

Dabei zeigte sich, dass keines der Peptide QRETPEGAEAKPWY, TKPIELGPDEPKAV und CGTKPIELGPDEPKAVC, obgleich von ihrer Primärsequenz verwandt mit den erfindungsgemäß vorgesehenen Peptiden zur Anwendung, irgendeine Wirkung auf den Natrium-Fluss hatte und somit auch keine aktivierende Wirkung auf den Amilorid-sensitiven epithelialen Natrium-Ionenkanal (ENaC) aufwies, wohingegen das erfindungsgemäße Peptid zur Anwendung eine Zunahme des Natrium-Stroms über die des Kontrollwertes induzierte, wenn es der Bad-Lösung in einem Ganzzell-Patch-Clamp-Test unter Verwendung von A549-Zellen zugesetzt wurde. Da somit die drei Vergleichspeptide keine Wirkung auf den Amilorid-sensitiven epithelialen Natrium-Ionenkanal (ENaC), verglichen mit der positiven Kontrolle (erfindungsgemäßes Peptid mit SEQ ID Nr. 1; CGQRETPEGAEAKPWYC) in einem Ganzzell-Patch-Clamp-Test unter Verwendung von A549-Zellen zeigten, die Clearance von Alveolarflüssigkeit jedoch eine Konsequenz dieser Natrium-Ionenbewegung über die alveolären Epithelialzellen ist, kann geschlossen werden, dass diese Peptide gemäß Stand der Technik - im Gegensatz zum erfindungsgemäßen Peptid zur Anwendung - nicht in der Lage sind, Lungenödeme zu reduzieren, obgleich sowohl von den linearen als auch von den zyklischen Peptiden, die in der EP 2 009 023 A1 beschrieben werden, jeweils besonders bevorzugte Varianten im vorliegenden Beispiel untersucht wurden (QRETPEGAEAKPWY, TKPIELGPDEPKAV und CGTKPIELGPDEPKAVC, die als Peptide SEQ ID Nr. 18, 76 und SEQ ID Nr. 2 in der EP 2 009 023 A1 angegeben sind). Dies ist umso bemerkenswerter, als den Vergleichspeptiden in der EP 2 009 023 A1 eine Aktivität bei der Bekämpfung von Ödemen zugerechnet worden ist.

Dies zeigt einerseits, dass die erfindungsgemäß vorgesehenen Merkmale, insbesondere die Zyklisierung und die Kernsequenz TX₁EX₂X₃E bzw. TPEGAE, essentielle Merkmale der vorliegenden Erfindung sind. Andererseits begründen die vorliegenden Untersuchungen auch wissenschaftlich erhärtete Zweifel an der Annahme, dass diese Peptide selbst zu der im Stand der Technik vorgeschlagenen Ödembehandlung geeignet sind. Das vorliegende Beispiel, welches mit dem in der wissenschaftlichen Fachwelt anerkannten Testsystem des Ganzzell Patch-Clamp-Test durchgeführt wurde, zeigt nämlich, dass das in der EP 2 009 023 A1 verwendete Untersuchungssystem (der TEER-Test) offensichtlich nicht geeignet ist, diese Aktivität zu belegen.

### Einleitung:

Der Flüssigkeitshaushalt in der gesunden erwachsenen menschlichen Lunge hängt von Ionentransportmechanismen über das Lungenepithel ab, wobei die Beteiligung der Na⁺-Transporter bei der Clearance der Alveolarflüssigkeit gut dokumentiert ist. Insbesondere stellt dabei der Amilorid-sensitive epitheliale Na⁺-Kanal (ENaC) einen limitierenden Schritt für die Na⁺-Resorption über das Alveolarepithel dar und spielt die Schlüsselrolle bei der Flüssigkeits-Reabsorption in der Lunge. Da eine verbesserte Clearance der Alveolarflüssigkeit direkt zu einer verbesserten Prognose und Wiederherstellung im Falle eines Lungenödems führt, bietet die Verbesserung der ENaC-Aktivität eine vielversprechende therapeutische Option zur Behandlung von Lungenödemen.

Die europäische Patentanmeldung EP 2 009 023 A1 schlägt dazu Peptide, wie QRETPEGAEAKPWY, TKPIELGPDEPKAV und CGTKPIELGPDEPKAVC (dort als Peptide SEQ ID Nr.18, SEQ ID Nr.76 und SEQ ID Nr. 2 beschrieben) als neue Moleküle vor, die die Ansammlung von überschüssiger Flüssigkeit im Gewebe hemmen oder reduzieren sollen.

Gemäß der Patentanmeldung EP 2 009 023 A1 wurde der sogenannte transepitheliale elektrische Widerstands-(TEER) Test zum Screening von anti-Lungenödem-Wirkstoffkandidaten eingesetzt. Der "TEER-Test" ist kein etablierter Test zur Vorhersage der Flüssigkeits-Clearance bei Lungenödemen (dieser Test lässt sich in der einschlägigen wissenschaftlichen Literatur nicht finden und hat auch im Hinblick auf die zur Anwendung kommenden Zellen (Calu-3-Zellen) keine Relevanz in einem Modell zum Gasaustausch in der menschlichen Lunge). Im vorliegenden Beispiel wurden - neben dem erfindungsgemäßen Peptid - die Peptide QRETPEGAEAKPWY, TKPIELGPDEPKAV und (zyklisiertes) CGTKPIELGPDEPKAVC mit Hilfe eines Ganzzell-Patch-Clamp-Assays untersucht, einer etablierten Methodik zur Messung der Ionen-Bewegung über die Zellmembranen und speziell für das Messen des Natrium-Transports über die Zellmembran von Alveolarepithelzellen (Eaton et al., Fed. Proc. 45 (1986), 2707; Hamill et al., Pflugers Arch. 391 (1981), 85-100). Dabei sollte geprüft werden, ob die Peptide den Amilorid-sensitiven epithelialen Natrium-Strom in Lungenzellen aktivieren können oder nicht.

Der "TEER-Test", wie er in der EP 2 009 023 A1 beschrieben worden ist, verwendet Zellschichten aus Calu-3-Zellen. Allerdings sind Calu-3-Zellen Bronchialzellen. Bronchialzellen repräsentieren etwa 1% der Oberfläche der menschlichen Lunge für den Gasaustausch und stellen daher kein angemessenes Modell für Alveolarepithelzellen dar, die etwa 99% der Oberfläche der menschlichen Lunge für den Gasaustausch ausmachen. Im vorliegenden Beispiel wurden die menschliche Alveolarepithelzelllinie A549 verwendet, da diese Zelllinie den allgemein akzeptierten experimentellen Standard definiert und in der Literatur als das Modell der Wahl für Alveolarepithelzellen angesehen wird (Lazrak et al., Am. J. Physiol. Lung Cell. Mol. Physiol. 278 (2000), 848-857).

### Versuchsdurchführung

### Untersuchte Peptide

Peptid "AP301" (erfindungsgemäßes Peptid zur Anwendung):

Synthetisches Peptid QRETPEGAEAKPWY:
H-Gln-Arg-Glu-Thr-Pro-Glu-Gly-Ala-Glu-Ala-Lys-Pro-Trp-Tyr-OH (SEQ ID Nr.3, im Stand der Technik als geeignet zur Behandlung von Ödemen beschrieben, ist jedoch, im Gegensatz zur erfindungsgemäßen Form, bei diesem Experiment nicht zyklisiert)

Synthetisches Peptid TKPIELGPDEPKAV:
H-Thr-Lys-Pro-Ile-Glu-Leu-Gly-Pro-Asp-Glu-Pro-Lys-Ala-Val-OH (SEQ ID Nr. 16; im Stand der Technik als geeignet zur Behandlung von Ödemen beschrieben, ist jedoch im Gegensatz zu den erfindungsgemäßen Peptiden zur Anwendung nicht zyklisiert und enthält nicht die Kernsequenz TX₁EX₂X₃E bzw. TPEGAE)

Synthetisches Peptid CGTKPIELGPDEPKAVC:
Cyclo-H-Cys-Gly-Thr-Lys-Pro-Ile-Glu-Leu-Gly-Pro-Asp-Glu-Pro-Lys-Ala-Val-Cys-OH (SEQ ID Nr. 17; im Stand der Technik als geeignet zur Behandlung von Ödemen beschrieben, enthält jedoch im Gegensatz zu den erfindungsgemäßen Peptiden zur Anwendung nicht die Kernsequenz TX₁EX₂X₃E bzw. TPEGAE)

### Peptidsynthese

Alle Peptide im vorliegenden Beispiel wurden durch Festphasen-Peptidsynthese nach der Fluorenylmethyloxycar-bonyl/t-Butyl-Schutz-Strategie auf 2-Chlorotritylchlorid-Harz hergestellt. Diisopropylcarbodiimid und N-Hydroxybenzotriazol wurden als Kopplungsreagenzien eingesetzt. Alle Kopplungsschritte wurden in N-N-Dimethylformamid durchgeführt. Geschützte Aminosäuren wurden nacheinander an die Peptidkette gekoppelt, beginnend mit der C-terminalen Aminosäure. Entschützen des Fluorenylmethoxycarbonyl wurde in 20% Piperidin in N-N-Dimethylformamid durchgeführt. Spaltung des vervollständigten, teilweise geschützten Peptids vom Harz wurde in einer 1:1-Mischung aus Essigsäure und Dichlormethan durchgeführt.

Im Falle des Peptids SEQ ID Nr. 1, wurde nach Spaltung vom Harz die Seitenkette-Entschützung in 95% Trifluoressigsäure, 5% Wasser, durchgeführt, gefolgt von Cyclisierung des linearen Rohpeptids durch Oxidation der terminalen Cysteinreste durch Sauerstoffzufuhr (O₂ bei 1,2 bar) bei pH 8,5 für ungefähr 100 Stunden.

Das Rohpeptid-Produkt wurde durch Umkehrphasen-Mitteldruck-Flüssigkeits-Chromatographie (RP-MPLC) auf einer RP-C18-Kieselgelsäule mit einem Gradienten von 5% - 40% Acetonitril gereinigt. Schließlich wurde das Trifluoracetat-Gegenion durch Acetat auf einer Lewatit MP64-Säule ersetzt (Acetat-Form). Nach einem letzten Waschschritt in Wasser wurde das gereinigte Peptid als Acetatsalz lyophilisiert und als weißes bis cremefarbenes Pulver erhalten. Im Fall von Peptid 2 bereitete die intramolekulare Disulfidbrücke Probleme beim Ablösen von der Lewatit-Säule, daher wurde dieses zyklische Peptid in der Trifluoracetat- anstatt der Acetat-Form verwendet.

### Charakterisierung der Peptide

Die molekularen Massen der Peptide wurden durch Elektrospray-Ionisierungs-Massenspektrometrie oder MALDI-TOF-MS bestätigt; die Reinheit wurde durch analytische Hochleistungs-Flüssigkeitschromatographie ermittelt.

Die Peptide wurden bei -20°C gelagert.

### Patch-Clamp-Protokoll

Der Ganzzell-Patch-Clamp-Test unter Verwendung von A549-Zellen erfolgte wie in Hazemi et al. beschrieben (J. Med. Chem. 53 (2010), 8021-8029). Lösungen der Peptide wurden der externen (Bad)-Lösung im Patch-Clamp-Test hinzugefügt, so dass eine Endkonzentration von 300 nM erreicht wurde. In Fällen, wo eine Erhöhung des Stromes nach Zugabe eines gegebenen Peptids beobachtet wurde, wurde - nachdem der Strom einen stationären Zustand erreichte - der Badlösung eine Amilorid-Lösung (auf 100 mM Endkonzentration) zugesetzt, um den Amilorid-sensitiven vom Amilorid-insensitiven Strom zu unterscheiden. Der Amilorid-sensitive Strom wurde dann durch Subtrahieren des Strom-Wertes nach Zugabe von Amilorid (Amilorid-insensitiv) vom Strom-Wert des stationären Zustandes vor der Zugabe von Amilorid berechnet. Für jedes Peptid wurden drei Experimente in verschiedenen A549 Zellen (n = 3) durchgeführt.

### Ergebnisse

Das erfindungsgemäßes Peptid zur Anwendung; SEQ ID Nr. 1 ("AP301"); positives Kontroll-Peptid) führte, wenn es der Badlösung in einem Ganzzell Patch-Clamp-Test unter Verwendung von A549-Zellen in einer Endkonzentration von 240 nM zugesetzt wurde, zu einer Erhöhung des aktiven Na⁺-Stroms von einem Kontrollwert von 86 pA ± 5 pA (vor Zugabe von AP301) auf ein Maximum von 1073 ± 15 pA (nach Zugabe von AP301). Die nachfolgende Zugabe von Amilorid verursachte einen Rückfall des Stromes auf 36 pA ± 5 pA. Dies zeigte, dass der Strom, der durch AP301 erhöht worden ist, der Amilorid-sensitive Na⁺-Strom ist (Figur 4).

Wenn die drei synthetischen Vergleichspeptide, QRETPEGAEAKPWY, TKPIELGPDEPKAV und CGTKPIELGPDEPKAVC in separaten Ganzzell-Patch-Clamp-Experimenten unter Verwendung von A549-Zellen der Badlösung in einer Endkonzentration von 300 nM hinzugefügt wurden, konnte keine Auswirkung auf den Strom beobachtet werden: die Werte blieben im Bereich des Kontrollwertes (Figuren 5-7).

### Diskussion der Ergebnisse

Im vorliegenden Beispiel wurde die Fähigkeit des Peptids gemäß der vorliegenden Erfindung ("AP301") als positive Kontrolle bei der Erhöhung des Amilorid-sensitiven Na⁺-Stroms in einem Ganzzell-Patch-Clamp-Test mit A549 Zellen gezeigt. Zugabe von AP301 zur Badlösung führte zu einer Erhöhung des Stromes ausgehend von einem Kontrollwert von 86 pA ± 5 pA (vor der Zugabe von AP301) auf ein Maximum von 1073 ± 15 pA (nach Zugabe von AP301). Die anschließende Zugabe von Amilorid verursachte einen Rückfall auf 36 pA ± 5 pA. Dies zeigt, dass AP301 den Amilorid-sensitiven Na⁺-Strom von 50 pA auf 1037 pA erhöht und somit die aktivierende Wirkung von AP301 auf den Amilorid-sensitiven epithelialen Na⁺-Kanals (ENaC) bestätigt (vgl. auch Tzotzos et al., Pulm. Pharmacol. Ther. 26 (2013), 356-363), der in der Lunge apikal in Alveolarepithelzellen angeordnet ist. Aktivierung von ENaC führt zu einer Erhöhung der Na⁺-Aufnahme aus der alveolären Flüssigkeit in die Epithelschicht, so dass die osmotische Triebkraft erhöht wird, die die Clearance der Alveolarflüssigkeit untermauert und dazu führt, dass Wasser aus den Alveoli in die Interstitialschicht unter dem Epithel fließt. Darauf könnte der Mechanismus beruhen, der dem beobachteten Alveolarflüssigkeits-Clearingeffekt von direkt an die Lunge verabreichtem AP301 zugrunde liegt.

Jedes der drei anderen synthetischen Vergleichspeptide, QRETPEGAEAKPWY, TKPIELGPDEPKAV und CGTKPIELGPDEPKAVC, wurde ebenfalls auf die Fähigkeit getestet, den Na⁺-Strom zu beeinflussen, wenn es der Badlösung in einem Ganzzell-Patch-Clamp-Test mit A549-Zellen hinzugefügt wurde. Doch anders als AP301, welches eine sofortige verstärkende Wirkung zeigte, hatte keines der drei anderen Peptide einen Einfluss auf den Strom in diesen Zellen, selbst in einer etwas höheren Anwendungskonzentration als das erfindungsgemäße Peptid zur Anwendung AP301 (300 nM für die drei Peptide, 240 nM für AP301).

### Beispiel 4: Aktivierung des Amilorid-sensitiven Natrium-Ionenkanals (ENaC) durch die erfindungsgemäßen Peptide zur Anwendung

Die erfindungsgemäßen Peptide zur Anwendung SEQ ID Nrn. 1 und 11 bis 15 wurden in Zell-basierten Studien umfassend charakterisiert. Diese zyklischen Peptide SEQ ID Nrn. 1 und 11 bis 15 aktivieren in Lungenzellen den Amilorid-sensitiven Natrium-Ionenkanal (ENaC). Dadurch wird die Gleichwertigkeit dieser Peptide mit dem zuvor untersuchten AP301 in der Wirkung gemäß der vorliegenden Erfindung verdeutlicht.

### Peptidsequenzen

SEQ ID Nr. 1: CGQRETPEGAEAKPWYC: Die Zyklisierung des Peptids wurde dadurch erreicht, dass die terminalen Cysteine (C) unter Herausbildung einer Schwefelbrücke oxidiert wurden. SEQ ID Nr. 11: KSPGQRETPEGAEAKPWYE: Im zyklischen Peptid SEQ ID Nr. 11 sind die Aminosäuren von der C-terminalen Aminosäure Glutaminsäure (E) bis zur N-terminalen Aminosäure Lysin (K) peptidisch verknüpft, während die N-terminale Aminosäure Lysin (K) mit der C-terminalen Aminosäure Glutaminsäure (E) mit Hilfe einer Amidbindung zwischen dem Stickstoff der epsilon-Aminogruppe der Seitenkette des Lysins und dem gamma-Kohlenstoff in der Seitengruppe der Glutaminsäure verbunden ist.

SEQ ID Nr. 12: KGQRETPEGAEAKPWYG: Im zyklischen Peptid SEQ ID Nr. 12 sind die Aminosäuren von der C-terminalen Aminosäure Glycin (G) bis zur N-terminalen Aminosäure Lysin (K) peptidisch verknüpft, während die N-terminale Aminosäure Lysin (K) mit der C-terminalen Aminosäure Glycin (G) mit Hilfe einer Amidbindung zwischen dem Stickstoff der epsilon-Aminogruppe der Seitenkette des Lysins und dem Kohlenstoff der Karboxylgruppe des Glycins verbunden ist.

SEQ ID Nr. 13: Ornithin-GQRETPEGAEAKPWYG: Im zyklischen Peptid SEQ ID Nr. 13 sind die Aminosäuren von der C-terminalen Aminosäure Glycin (G) bis zur N-terminale Aminosäure Ornithin (Orn) peptidisch verknüpft, während die N-terminale Aminosäure Ornithin (Orn) mit der C-terminalen Aminosäure Glycin (G) mit Hilfe einer Amidbindung zwischen dem Stickstoff der delta-Aminogruppe der Seitenkette des Ornithins und dem Kohlenstoff der Karboxylgruppe des Glycins verbunden ist.

SEQ ID Nr. 14: 4-Aminobutansäure-GQRETPEGAEAKPWYD: Im zyklischen Peptid SEQ ID Nr. 14 sind die Aminosäuren von der C-terminalen Asparaginsäure (D) bis zur N-terminalen Aminosäure Glycin (G) peptidisch verknüpft, während die C-terminale Asparaginsäure (D) mit der N-terminalen Aminosäure Glycin mit Hilfe einer Amidbindung zwischen dem Stickstoff der Aminogruppe des N-terminalen Glycin und dem Kohlenstoff C1 der Karboxylgruppe der 4-Aminobuttersäure einerseits, und mit Hilfe einer Amidbindung zwischen dem Stickstoff der Aminogruppe der 4-Aminobuttersäure und dem Kohlenstoff der Karboxylgruppe der Seitenkette der C-terminalen Asparaginsäure andererseits, verbunden ist.

SEQ ID Nr. 15: β-Alanin-GQRETPEGAEAKPWYE: Im zyklischen Peptid SEQ ID Nr. 15 sind die Aminosäuren von der C-terminalen Glutaminsäure (E) bis zur N-terminalen Aminosäure Glycin (G) peptidisch verknüpft, während die C-terminale Glutaminsäure (E) mit der N-terminalen Aminosäure Glycin mit Hilfe einer Amidbindung zwischen dem Stickstoff der Aminogruppe des N-terminalen Glycins und dem Kohlenstoff C1 der Karboxylgruppe des β-Alanins einerseits, und mit Hilfe einer Amidbindung zwischen dem Stickstoff der Aminogruppe des β-Alanins und dem Kohlenstoff der Karboxylgruppe der Seitenkette der C-terminalen Glutaminsäure andererseits verbunden ist.

SEQ ID Nr. 19: CGQREAPAGAAAKPWYC (nicht erfindungsgemäß): Die Zyklisierung des Peptid SEQ ID Nr. 19 wurde dadurch erreicht, dass die terminalen Cysteine (C) unter Herausbildung einer Schwefelbrücke oxidiert wurden.

### Peptidsynthese

Die zyklischen Peptide SEQ ID Nrn. 1, 11 bis 15 und 19 wurden mittels Fmoc-Festphasensynthese vollautomatisch unter Einhaltung folgender Schritte hergestellt: Sequenzielle Kopplung der Aminosäuren; Selektive Abspaltung von der Festphase; Reinigung und Lyophilisation, Selektive Zyklisierung; Abspaltung der Schutzgruppen; Reinigung und Lyophilisation; Analytische Untersuchung.

Anschließend wurden die zyklischen Peptide SEQ ID Nrn. 1 und 11 bis 15 (erfindungsgemäß) sowie 19 (nicht erfindungsgemäß) mittels Reverser HPLC auf Reinheit und Masse untersucht.

Die Reinheit des zyklischen Peptids SEQ ID Nr. 1 war 96.3%. m/z (ESI) 1924.2 (M++1). Die Reinheit des zyklischen Peptids SEQ ID Nr. 11 war 96.3%. m/z (ESI) 1924.2 (M++1). Die Reinheit des zyklischen Peptids SEQ ID Nr. 12 war 98.8%. m/z (ESI) 1888.2 (M++1). Die Reinheit des zyklischen Peptids SEQ ID Nr. 13 war 97.4%. m/z (ESI) 1873.4 (M++1). Die Reinheit des zyklischen Peptids SEQ ID Nr. 14 war 99%. m/z (MALDI-TOF) 1901.6 (M++1). Die Reinheit des zyklischen Proteins SEQ ID Nr. 15 war 99%. m/z (MALDI-TOF) 1902.7 (M++1). Die Reinheit des zyklischen Peptids SEQ ID Nr. 19 war 95%. m/z (MALDI-TOF) 1778.02 (M++1).

Alle erfindungsgemäßen Peptide SEQ ID Nrn. 1 und 11 bis 15 haben folgende gemeinsame strukturelle Eigenschaft:
Sequenz: X₁-GQRETPEGAEAKPWY-X₂,
wobei X₁ eine Aminosäure oder 1 bis 4 Aminosäuren, insbesondere 1 oder 3 Aminosäuren darstellt, wobei die Aminosäuren natürliche oder unnatürliche Aminosäuren sind,
wobei X₁ die Aminosäure C, K, Ornithin, 4-Amino Buttersäure, β-Alanin, oder die Sequenz KSP darstellt,
wobei X₂ eine natürliche oder unnatürliche Aminosäure sein kann,
wobei X₂ die Aminosäure C, D, G oder E sein kann,
und wobei X₁ die N-terminale Aminosäure und X₂ die C-terminale Aminosäure sind.

### Elektrophysiologische Untersuchungen des Amilorid-sensitiven Natrium-Ionenkanal (ENaC)

Makroskopische Natrium-Ionenströme wurden von menschlichen Lungenepithelzellen A549 mit der "whole cell" Konfiguration mittels "Patch-clamp" Technik (Hamill et al, Pflugers Arch. 391 (1981), 85-100) abgeleitet. Für die Stromableitungen in der "whole cell" Konfiguration wurden folgende Bad- und Elektrodenlösungen verwendet:
Badlösung: 135 mM Natrium Methansulfonat, 10 mM NaCl, 2.7 mM KCl, 1.8 mM CaCl₂, 2 mM MgCl₂, 5.5 mM Glucose, und 10 mM HEPES, pH 7.4.
Elektrodenlösung: 120 mM Kaliummethansulfonat, 15 mM KCl, 6 mM NaCl, 1 mM Mg₂ATP, 2 mM Na3ATP, 10 mM HEPES, and 0.5 mM EGTA (pH 7.2).

Deckgläser mit den darauf kultivierten Zellen wurden in ein 1 ml fassendes Versuchsbad transferiert, auf dem Mikroskoptisch (Axiovert 100, 400-fache Vergrößerung) fixiert und die Zellen mit der oben beschriebenen Badlösung superfundiert. Sodann wurde von einer geeigneten Zelle (welche am Deckglas haftet) der Strom abgeleitet. Dazu wurde eine mit einer Elektrolytlösung gefüllte Mikroelektrode (Glaskapillare mit einer definierten, Hitze-polierten Spitzenöffnung von ca. 1-3 µm, entspricht einem Widerstand der Elektrodenspitze von 3-5 MΩ) auf die Zelle aufgesetzt und die Membran angesaugt, sodass ein "Gigaohm-Seal" zwischen Membran und Elektrode gebildet wurde, um den Leckstrom zu minimieren. Bei der "whole cell"-Konfiguration wurde die Membran unter der Elektrodenspitze durchbrochen, damit der Strom, der durch alle Ionenkanäle der Zelle fließt, gemessen werden kann. Bei Erhalt eines "Gigaohm-Seals" wurde über einen Vorverstärker (CV-4 Headstage, Axon Instruments) und Verstärker (Axopatch 1D, Axon Instr.) ein definiertes Membranhaltepotential angelegt und der Strom, der dabei durch die Ionenkanäle fließt, gemessen.

Das Pulsprotokoll bestand aus einer Hyperpolarisation der Zellmembran auf -100 mV für 5 s und darauf folgender schrittweiser Depolarisation in 20 mV Schritten auf +100 mV.

Dieses Protokoll wurde vor (Kontrolle) und nach Zugabe der zyklischen Proteine durchgeführt. Die so erhaltenen Stromableitungen wurden gespeichert und mit Hilfe des Programms PCLAMP 6.0 analysiert. Dazu wurden die unter Anwesenheit von Amilorid erhaltenen Stromableitungen von den vorher registrierten Strömen subtrahiert, sodass der Amilorid-sensitive Natrium-Strom durch die epithelialen Natrium-Kanäle ermittelt werden konnte.

Die Ergebnisse der Messungen sind in der Tabelle 1 zusammengefasst. Die Aktivität der einzelnen Peptide ist als EC50 (in nM) angegeben. Die EC50 ist die effektive Konzentration, bei der 50% von der maximalen Aktivität (d.h. maximale Erhöhung der Stromstärke, I) gemessen wird.

Tabelle 1. Aktivität der erfindungsgemäßen Peptide zur Anwendung SEQ ID 1 und SEQ ID 11 - 15, sowie des nicht erfindungsgemäßen Peptids zur Anwendung SEQ ID Nr. 19 auf den zellularen Amilorid-sensitiven Natrium-Ionenstrom. Die Aktivität wird als effektive Konzentration bei 50% der Maximalaktivität (EC₅₀) angegeben.

| **Zyklisches Peptid** | **EC₅₀ (nM)** |
|---|---|
| | |
| SEQ ID Nr. 1 | 54 |
| SEQ ID Nr. 11 | 56 |
| SEQ ID Nr. 12 | 38 |
| SEQ ID Nr. 13 | 45 |
| SEQ ID Nr. 14 | 24 |
| SEQ ID Nr. 15 | 19 |
| SEQ ID Nr. 19 | keine Aktivität |

Die Aktivität der zyklischen Peptide SEQ ID Nrn. 1 und 11 bis 15 in Abhängigkeit der Konzentration ist in der Figur 8 dargestellt. Die maximale Aktivität wurde mit 100% angegeben.

Die dargestellten Untersuchungen zeigen, dass die erfindungsgemäßen Peptide zur Anwendung SEQ ID Nrn. 1 und 11 bis 15 biologisch aktiv sind, wohingegen das nichterfindungsgemäße Peptid SEQ ID Nr. 19 nicht aktiv ist. Der Unterschied zwischen den zyklischen Peptiden SEQ ID Nrn. 1 und 11 bis 15 und dem zyklischen Peptid SEQ ID Nr. 19 besteht darin, dass innerhalb der allgemeinen Peptidsequenz X₁-GQRETPEGAEAKPWY-X₂ die Aminosäure T (an 5. Position) und die Aminosäure E (an 7. Position) und die Aminosäure E (an 10. Position) durch Alanin ausgetauscht wurden. Wesentlich ist also die Sequenz TPEGAE. Die Struktur von X₁ sowie X₂ haben keinen wesentlichen Einfluss auf die Aktivität.

Zusammenfassung der Sequenzen:
SEQ ID Nr. 1 CGQRETPEGAEAKPWYC
SEQ ID Nr. 2 TPEGAE
SEQ ID Nr. 3 QRETPEGAEAKPWY
SEQ ID Nr. 4 PKDTPEGAELKPWY
SEQ ID Nr. 5 CGPKDTPEGAELKPWYC
SEQ ID Nr. 6 CGQKETPEGAEAKPWYC
SEQ ID Nr. 7 CGQRETPEGAEARPWYC
SEQ ID Nr. 8 CGQRETPEGAEAKPC
SEQ ID Nr. 9 CQRETPEGAEAKPWYC
SEQ ID Nr. 10 CGQRETPEGAEAKFWYC
SEQ ID Nr. 11 KSPGQRETPEGAEAKPWYE
SEQ ID Nr. 12 KGQRETPEGAEAKPWYG
SEQ ID Nr. 13 Ornithin-GQRETPEGAEAKPWYG
SEQ ID Nr. 14 4-Aminobutansäure-GQRETPEGAEAKPWYD
SEQ ID Nr. 15 β-Alanin-GQRETPEGAEAKPWYE
SEQ ID Nr. 16 TKPIELGPDEPKAV
SEQ ID Nr. 17 CGTKPIELGPDEPKAVC
SEQ ID Nr. 18 GQRETPEGAEAKPWY
SEQ ID Nr. 19 CGQREAPAGAAAKPWYC
SEQ ID Nr. 20 TXEXXE

## Patentansprüche

1. Peptid, welches aus 7-20, insbesondere 7-17 benachbarten Aminosäuren besteht und das Hexamer TX₁EX₂X₃E umfasst, wobei X₁, X₂ und X₃ jede natürliche oder nicht natürliche Aminosäure sein kann, wobei das Peptid keine TNF-Rezeptor-Bindungsaktivität aufweist und zyklisiert ist, zur Anwendung zur Behandlung und Vermeidung der pulmonalen Form der Höhenkrankheit.

2. Peptid zur Anwendung nach Anspruch 1, wobei das Peptid aus 7-20, insbesondere 7-17 benachbarten Aminosäuren besteht und das Hexamer TPEGAE umfasst.

3. Peptid zur Anwendung nach Anspruch 1 oder 2, wobei das zyklisierte Peptid aus einer Sequenz aufeinander folgender Aminosäuren, ausgewählt aus der Gruppe bestehend aus CGQRETPEGAEAKPWYC, CGPKDTPEGAELKPWYC, CGQKETPEGAEAKPWYC, CGQKETPEGAEAKPWYC, CGQRETPEGAEARPWYC, CGQRETPEGAEAKPC, CQRETPEGAEAKPWYC, CGQRETPEGAEAKFWYC, KSPGQRETPEGAEAKPWYE, KGQRETPEGAEAKPWYG, Ornithin-GQRETPEGAEAKPWYG), 4-Aminobutansäure-GQRETPEGAEAKPWYD, β-Alanin-GQRETPEGAEAKPWYE und Fragmenten von mindestens 7 Aminosäuren davon, welche Fragmente das Hexamer TPEGAE aufweisen, besteht.

4. Peptid zur Anwendung nach einem der Ansprüche 1 bis 3, **dadurch gekennzeichnet, dass** das Peptid die Aminosäuresequenz CGQRETPEGAEAKPWYC umfasst und über die C-Reste zyklisiert ist.

5. Peptid zur Anwendung nach einem der Ansprüche 1 bis 4, **dadurch gekennzeichnet, dass** das Peptid durch eine Disulfidbrücke zwischen den C-Resten zyklisiert ist.

6. Pharmazeutische Zusammensetzung zur Anwendung zur Behandlung und Vermeidung der pulmonalen Form der Höhenkrankheit, enthaltend ein Peptid wie in einem der Ansprüche 1 bis 5 definiert, **dadurch gekennzeichnet, dass** es in einer pharmazeutischen Zusammensetzung formuliert ist, die zur Verabreichung am Menschen geeignet ist, und einen pharmazeutisch akzeptablen Träger umfasst.

7. Pharmazeutische Zusammensetzung zur Anwendung nach Anspruch 6, **dadurch gekennzeichnet, dass** sie einen pharmazeutisch akzeptablen Träger umfasst, der ausgewählt ist aus Wasser, insbesondere Wasser für Injektion, Kochsalz, Natriumphosphat, Natriumacetat, Natriumcarbonat, Zitrat, Glycin, Glycylglycin, Histidin, Lysin, Arginin, TRIS, Natriumcitrat, Ringer-Lösung, Dextrose, Mannit, Trehalose, Saccharose, Sorbit, Fruchtzucker, Maltose, Lactose oder Dextran, Hank-Lösung, fixierte Öle, Ethyloleat, Substanzen, die die Isotonie und die chemische Stabilität verbessern, Konservierungsmittel, pharmazeutisch akzeptable Proteine, Polysaccharide, Polymilchsäuren, Polyglykolsäuren, polymere Aminosäuren und Aminosäure-Copolymere.

8. Pharmazeutische Zusammensetzung zur Anwendung nach Anspruch 6 oder 7, **dadurch gekennzeichnet, dass** sie das Peptid in einer Menge von 1 µg bis 10 g, vorzugsweise von 10 µg bis 1 g, insbesondere von 1 mg bis 100 mg, umfasst.

9. Pharmazeutische Zusammensetzung zur Anwendung nach einem der Ansprüche 6 bis 8, **dadurch gekennzeichnet, dass** sie in flüssiger Form vorliegt und das Peptid in einer Menge von 1 µg bis 10 g, vorzugsweise von 10 µg bis 1 g, insbesondere von 1 mg bis 100 mg, umfasst und in einem Volumen von 0,5 bis 10 ml, insbesondere in einem Volumen von 1 bis 5 ml, vorliegt.

10. Pharmazeutische Zusammensetzung zur Anwendung nach einem der Ansprüche 6 bis 9, **dadurch gekennzeichnet, dass** das Peptid in einer vernebelbaren Pulverformulierung oder in einer vernebelbaren Flüssigformulierung vorliegt.

## Claims

1. A peptide which consists of 7-20, in particular 7-17 adjacent amino acids and comprises the hexamer TX₁EX₂XE, wherein X₁, X₂ and X₃ may be any natural or unnatural amino acid, wherein the peptide does not exhibit TNF receptor binding activity and is cyclized, for use in the treatment and prevention of the pulmonary form of altitude sickness.

2. The peptide for use as claimed in claim 1, wherein the peptide consists of 7-20, in particular 7-17, adjacent amino acids and comprises the hexamer TPEGAE.

3. The peptide for use as claimed in claim 1 or claim 2, wherein the cyclized peptide consists of a sequence of consecutive amino acids selected from the group consisting of CGQRETPEGAEAKPWYC, CGPKDTPEGAELKPWYC, CGQKETPEGAEAKPWYC, CGQKETPEGAEAKPWYC, CGQRETPEGAEARPWYC, CGQRETPEGAEAKPC, CQRETPEGAEAKPWYC, CGQRETPEGAEAKFWYC, KSPGQRETPEGAEAKPWYE, KGQRETPEGAEAKPWYG, ornithine-GQRETPEGAEAKPWYG, 4-aminobutanoic acid-GQRETPEGAEAKPWYD, β-alanine-GQRETPEGAEARPWYE and fragments of at least 7 amino acids thereof, which fragments have the hexamer TPEGAE.

4. The peptide for use as claimed in one of claims 1 to 3, **characterized in that** the peptide comprises the amino acid sequence CGQRETPEGAEAKPWYC and is cyclized via the C residues.

5. The peptide for use as claimed in one of claims 1 to 4, **characterized in that** the peptide is cyclized by a disulphide bridge between the C residues.

6. A pharmaceutical composition for use for the treatment and prevention of the pulmonary form of altitude sickness, containing a peptide as defined in one of claims 1 to 5, **characterized in that** it is formulated in a pharmaceutical composition which is suitable for administration to humans, and comprises a pharmaceutically acceptable support.

7. The pharmaceutical composition for use as claimed in claim 6, **characterized in that** it comprises a pharmaceutically acceptable support which is selected from water, in particular water for injection, common salt, sodium phosphate, sodium acetate, sodium carbonate, citrate, glycine, glycyl glycine, histidine, lysine, arginine, TRIS, sodium citrate, Ringer's solution, dextrose, mannitol, trehalose, saccharose, sorbitol, fructose, maltose, lactose or dextran, Hank's solution, fixed oils, ethyl oleate, substances which improve isotonicity and chemical stability, preservatives, pharmaceutically acceptable proteins, polysaccharides, polylactic acids, polyglycolic acids, polymeric amino acids and amino acid copolymers.

8. The pharmaceutical composition for use as claimed in claim 6 or claim 7, **characterized in that** it comprises the peptide in a quantity of 1 µg to 10 g, preferably 10 µg to 1 g, in particular 1 mg to 100 mg.

9. The pharmaceutical composition for use as claimed in one of claims 6 to 8, **characterized in that** it is in the liquid form and comprises the peptide in a quantity of 1 µg to 10 g, preferably 10 µg to 1 g, in particular 1 mg to 100 mg and is in a volume of 0.5 to 10 mL, in particular in a volume of 1 to 5 mL.

10. The pharmaceutical composition for use as claimed in one of claims 6 to 9, **characterized in that** the peptide is in the form of a nebulisable powder formulation or a nebulisable liquid formulation.

## Revendications

1. Peptide qui est constitué de 7-20, en particulier de 7-17 acides aminés voisins, et qui comprend l'hexamère TX₁EX₂X₃E, X₁, X₂ et X₃ pouvant être tout acide aminé naturel ou non naturel, le peptide ne présentant pas d'activité de liaison au récepteur du TNF et étant cyclisé, pour une utilisation pour le traitement et la prévention de la forme pulmonaire du mal des montagnes.

2. Peptide pour l'utilisation selon la revendication 1, le peptide étant constitué de 7-20, en particulier de 7-17 acides aminés voisins, et comprenant l'hexamère TPEGAE.

3. Peptide pour l'utilisation selon la revendication 1 ou 2, le peptide cyclisé étant constitué d'une séquence d'acides aminés se suivant les uns des autres, choisis dans le groupe consistant en CGQRETPEGAEAKPWYC, CGPKDTPEGAELKPWYC, CGQKETPEGAEAKPWYC, CGQKETPEGAEAKPWYC, CGQRETPEGAEARPWYC, CGQRETPEGAEAKPC, CQRETPEGAEAKPWYC, CGQRETPEGAEAKFWYC, KSPGQRETPEGAEAKPWYE, KGQRETPEGAEAKPWYG, Ornithine-GQRETPEGAEAKPWYG, acide 4-aminobutyrique-GQRETPEGAEAKPWYD, β-alanine-GQRETPEGAEAKPWYE et les fragments d'au moins 7 acides aminés de ces dernières, qui comprennent des fragments de l'hexamère TPEGAE.

4. Peptide pour l'utilisation selon l'une des revendications 1 à 3, **caractérisé en ce que** le peptide comprend la séquence d'acides aminés CGQRETPEGAEAKPWYC et est cyclisé par l'intermédiaire des résidus C.

5. Peptide pour l'utilisation selon l'une des revendications 1 à 4, **caractérisé en ce que** le peptide est cyclisé par un pont disulfure entre les résidus C.

6. Composition pharmaceutique pour une utilisation pour le traitement et la prévention de la forme pulmonaire du mal des montagnes, contenant un peptide tel que défini dans l'une des revendications 1 à 5, **caractérisée en ce qu'**il est formulé dans une composition pharmaceutique qui convient à une administration à l'homme, et qui comprend un support pharmaceutiquement acceptable.

7. Composition pharmaceutique pour l'utilisation selon la revendication 6, **caractérisée en ce qu'**elle comprend un support pharmaceutiquement acceptable, qui est choisi parmi l'eau, en particulier l'eau p.p.i., le chlorure de sodium, le phosphate de sodium, l'acétate de sodium, le carbonate de sodium, le citrate, la glycine, la glycylglycine, l'histidine, la lysine, l'arginine, le TRIS, le citrate de sodium, la solution de Ringer, le dextrose, le mannitol, le tréhalose, le saccharose, le sorbitol, le fructose, le maltose, la lactose ou le dextran, la solution de Hank, les huiles fixes, l'oléate d'éthyle, les substances qui améliorent l'isotonie et la stabilité chimique, les conservateurs, les protéines pharmaceutiquement acceptables, les polysaccharides, les poly(acides lactiques), les poly(acides glycoliques), les acides aminés polymères et les copolymères d'acides aminés.

8. Composition pharmaceutique pour l'utilisation selon la revendication 6 ou 7, **caractérisée en ce qu'**elle comprend le peptide en une quantité de 1 µg à 10 g, de préférence de 10 µg à 1 g, en particulier de 1 mg à 100 mg.

9. Composition pharmaceutique pour l'utilisation selon l'une des revendications 6 à 8, **caractérisée en ce qu'**elle se présente sous forme liquide, et qu'elle comprend le peptide en une quantité de 1 µg à 10 g, de préférence de 10 µg à 1 g, en particulier de 1 mg à 100 mg, et est présent dans un volume de 0,5 à 10 ml, en particulier dans un volume de 1 à 5 ml.

10. Composition pharmaceutique pour l'utilisation selon l'une des revendications 6 à 9, **caractérisée en ce que** le peptide se présente dans une formulation poudrée nébulisable ou dans une formulation liquide nébulisable.
